(19) Europäisches Patentamt
European Patent Office
Office européen des brevets

(11) Publication number: **0 163 537**
**A1**

# (12) EUROPEAN PATENT APPLICATION

(21) Application number: **85303804.0**

(22) Date of filing: **30.05.85**

(51) Int. Cl.[4]: **C 07 D 295/10**
C 07 D 317/58, C 07 D 307/79
C 07 D 405/06, A 61 K 31/395

(30) Priority: **01.06.84 JP 110975/84**
**02.10.84 JP 205612/84**
**21.12.84 JP 268446/84**
**12.03.85 JP 47495/85**

(43) Date of publication of application:
**04.12.85 Bulletin 85/49**

(84) Designated Contracting States:
**AT BE CH DE FR GB IT LI LU NL SE**

(71) Applicant: **TOKYO TANABE COMPANY LIMITED**
**7-3 Nihonbashi-Honcho 2-chome**
**Chuo-ku Tokyo 103(JP)**

(72) Inventor: **Masazumi, Tomari**
**Imaiminami-cho Nakahara-ku**
**Kawasaki-shi Kanagawa 211(JP)**

(72) Inventor: **Masaki, Saeki Shinmatsudo Central**
**Parkhouse C-503 1 Shinmatsudo 5-chome**
**Matsudo-shi Chiba 270(JP)**

(72) Inventor: **Setsuo, Yamashita**
**Higashisayamagaoka 2-chome**
**Tokorozawa-shi Saitama 359(JP)**

(74) Representative: **Skailes, Humphrey John et al,**
**Frank B. Dehn & Co. Imperial House 15-19 Kingsway**
**London WC2B 6UZ(GB)**

## (54) 1-Propanone derivatives and pharmaceutical compositions containing same.

(57) 1-Propanone derivatives represented by the formula:

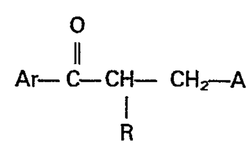

wherein Ar is a 4-cycloalkylphenyl, 3, 4-methylenedioxyphenyl or 2, 3-dihydro-5-benzofuranyl group, R is a straight-chain, branched or cyclic alkyl group having 1 to 6 carbon atoms or a cyclopentymethyl group, and A is an unsubstituted or substituted 1-pyrrolidinyl, piperidino, hexahydro-1*H*-azepin-1-y1 or octahydroazocin-1-y1 group, and physiologically acceptable acid addition salts thereof. These compounds are useful as central muscle relaxants.

EP 0 163 537 A1

# 1-PROPANONE DERIVATIVES AND PHARMACEUTICAL COMPOSITIONS CONTAINING SAME

## BACKGROUND OF THE INVENTION

(1) Field of the Invention

This invention relates to 1-propanone derivatives having central muscle relaxant activity. More particularly, it relates to compounds represented by the formula:

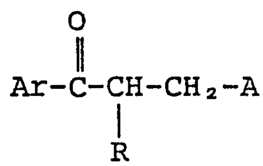

(I)

wherein Ar is a 4-cycloalkylphenyl, 3,4-methylenedioxyphenyl or 2,3-dihydro-5-benzofuranyl group, R is a straight-chain, branched or cyclic alkyl group having 1 to 6 carbon atoms or a cyclopentylmethyl group, and A is an unsubstituted or substituted 1-pyrrolidinyl, piperidino, hexahydro-1*H*-azepin-1-yl or octahydroazocin-1-yl group, and physiologically acceptable acid addition salts thereof.

(2) Description of the Prior Art

Conventionally, a number of 1-propanone derivatives having central muscle relaxant activity are known.

For example, 2-methyl-1-(4-methylphenyl)-3-(1-piperidinyl)-1-propanone [Tolperisone; cf. The Merck Index, 10th ed., 9351(1983)] is being widely used as a central muscle relaxant. As compounds having more excellent pharmacological activities than Tolperisone, 1-(4-$C_{2-3}$alkylphenyl)-2-methyl-3-(1-piperidinyl)-1-propanones are disclosed in U.S. Patents Nos. 3,995,047 and 4,181,803, and the compound in which the $C_{2-3}$alkyl group is ethyl (i.e., Eperisone) is described in The Merck Index, 10th ed., 3555(1983).

Moreover, a variety of 1-(4-substituted phenyl)-2-alkyl-3-(1-cyclic amino)-1-propanone compounds are known. In these compounds, however, the substituent of the 4-substituted phenyl group is hydroxy, lower alkyl, lower alkoxy, halogen, phenyl, amino, sulfonyl, carboxy, cyclohexylmethyl, cyclohexyloxy, cyclohexylthio or the like, and none of them have a cycloalkyl substituent.

Furthermore, a number of 1-(3,4-methylenedioxyphenyl)-3-(1-cyclic amino)-1-propanone compounds are known. However, none

of them have a substituent at the 2-position of 1-propanone. For example, 1-(3,4-methylenedioxyphenyl)-3-[1-piperidinyl(or 1-pyrrolidinyl)]-1-propanone as a urinary metabolite of safrole is disclosed in Biochim. Biophys. Acta., 230(2), 237-47(1971) [C.A., Vol. 76, 30698v(1972)], 1-(3,4-methylenedioxyphenyl)-3-(2-phenyl-1-pyrrolidinyl)-1-propanone exhibiting an adreno- and nicotinolytic action is disclosed in U.S.S.R. Patent No. 166348 [C.A., Vol. 62, 3995b(1965)], and 1-(3,4-methylenedioxyphenyl)-3-(1-piperidinyl)-1-propanone having antispasmodic activity is disclosed in J. Am. Chem. Soc., 71, 2048-50(1949).

In Yakugaku Zasshi, 97(5), 540-552 (1977), it is described that 1-(2,2-dimethyl-2,3-dihydro-5-benzofuranyl)-2-methyl-3-(1-piperidinyl)-1-propanone has antireserpine activity and exerts no influence on spontaneous motor movements.

## SUMMARY OF THE INVENTION

An object of the present invention is to provide novel 1-propanone derivatives represented by formula (I) and physiologically acceptable acid addition salts thereof which are useful as central muscle relaxants because of their anti-nicotine activity, anti-tremorine activity, muscle relaxant activity and rigidity reluxing effect. Another object of the present invention is to provide 1-propanone derivatives which are superior to Tolperisone and Eperisone in central muscle relaxant activity and duration of action.

## DETAILED DESCRIPTION OF THE PREFERRED EMBODIMENTS

In the compounds of the present invention which are within the scope of formula (I), the 4-cycloalkylphenyl group represented by Ar is 4-cyclopropylphenyl, 4-cyclobutylphenyl, 4-cyclopentylphenyl or 4-cyclohexylphenyl. The straight-chain, branched or cyclic alkyl group having 1 to 6 carbon atoms, which is represented by R, is methyl, ethyl, n-propyl, isopropyl, n-butyl, sec-butyl, isobutyl, tert-butyl, n-pentyl, n-hexyl, cyclopentyl, cyclohexyl or the like. The radical represented by A is 1-pyrrolidinyl, piperidino, hexahydro-

1*H*-azepin-1-yl or octahydroazocin-1-yl, and these radicals may have one or more substituents (such as hydroxy, lower alkyl, phenyl, phenylalkyl, carboxy, alkoxycarbonyl and the like) at any desired positions.

Since the compounds of the present invention have an asymmetric carbon atom at the 2-position, each of them has two optical isomers, i.e., the (+)- and (-)-isomers.

Physiologically acceptable acid addition salts of the compounds of the present invention include inorganic acid salts formed by reaction with hydrochloric acid, sulfuric acid, nitric acid, phosphoric acid, etc., and organic acid salts formed by reaction with acetic acid, citric acid, succinic acid, maleic acid, fumaric acid, tartaric acid, lactic acid, etc.

The compounds of the present invention can be prepared according to the following reaction equations:

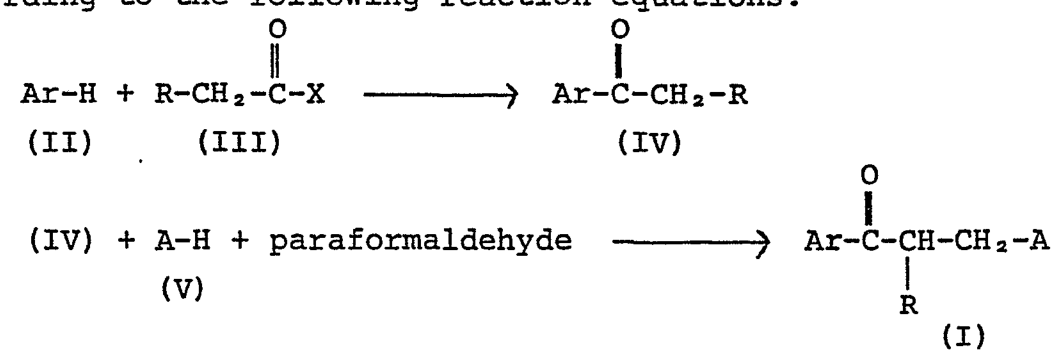

(II) (III) (IV)

(IV) + A-H + paraformaldehyde

(V)

(I)

wherein Ar, R and A have the meanings defined above and X is a halogen atom.

Specifically, a 4-cycloalkylbenzene, 3,4-methylenedioxybenzene or 2,3-dihydrobenzofuran of formula (II) and a fatty acid halide of formula (III) are subjected to a Friedel-Crafts reaction in the presence of a Lewis acid such as aluminum chloride or tin(IV) chloride. This reaction is carried out at a temperature of -75 to 100°C for a period of 10 minutes to 6 hours to obtain a 4'-cycloalkylphenone compound, 3',4'-methylenedioxyphenone compound or 2,3-dihydro-5-benzofuranyl-ketone compound of formula (IV). A fatty acid anhydride may be used in place of the fatty acid halide (III). As the reaction solvent, there may be used chloroform, carbon tetrachloride, methylene chloride, carbon disulfide, nitrobenzene and the like.

Then, according to the Mannich reaction, the compound (IV) so formed is reacted with an acid addition salt of a cyclic amine

(V) in the presence of paraformaldehyde to obtain a compound of formula (I) in the form of an acid addition salt. In order to accelerate the reaction, a small amount of an acid such as concentrated hydrochloric acid, concentrated sulfuric acid or the like may be added to the reaction system. The reaction is carried out at a temperature of 10 to 150°C for a period of 5 minutes to 15 hours. As the reaction solvent, methanol, ethanol, propanol, isopropanol, butanol, sec-butanol, tert-butanol, amyl alcohol, benzene, toluene, xylene and the like may be used alone or in admixture.

The desired compound of formula (I) can be obtained by treating the resulting acid addition salt with alkali. If necessary, this compound may be treated with a suitable acid to form a desired acid addition salt thereof.

Optical resolution of the compounds of the present invention can be carried out according to the fractional crystallization method in which a compound to be resolved is reacted with an optically active N-acetyl-α-phenylglycine and the resulting two diastereoisomers are separated on the basis of their difference in solubility in solvents.

Specifically, a (±)-compound of formula (I) is reacted with (-)-N-acetyl-D-α-phenylglycine to form the (-)-N-acetyl-D-α-phenylglycine salts of its (+)- and (-)-isomers. These salts are crystallized with and/or recrystallized from a suitable solvent mixture (such as acetone-isopropyl ether, acetone-ethyl ether, etc.) or a suitable solvent (such as ethyl acetate, etc.) to isolate and purify the slightly soluble (-)-N-acetyl-D-α-phenylglycine salt of the (+)-isomer. The resulting salt is treated with alkali to obtain the (+)-isomer of the compound of formula (I), which may be converted into an acid addition salt, if desired.

Then, the crystallization and/or recrystallization mother liquors obtained in the above-described procedure and containing a large amount of the easily soluble (-)-N-acetyl-D-α-phenylglycine salt of the (-)-isomer are concentrated under reduced pressure. The resulting residue is treated with an aqueous alkaline solution to obtain the free (-)-isomer, which is reacted with (+)-N-acetyl-L-α-phenylglycine to obtain a product composed mainly of the slightly soluble (+)-N-acetyl-

L-α-phenylglycine salt of the (-)-isomer. This product can be crystallized with and/or recrystallized from a suitable solvent mixture (such as acetone-isopropyl ether, acetone-ethyl ether, etc.) or a suitable solvent (such as ethyl acetate, etc.) to isolate and purify the slightly soluble (+)-N-acetyl-L-α-phenylglycine salt of the (-)-isomer. The resulting salt is treated in the same manner as described above for the (+)-isomer to obtain the (-)-isomer of the compound of formula (I), which may be converted into an acid addition salt, if desired.

Alternatively, the (±)-compound of formula (I) may be reacted with (-)-N-acetyl-L-α-phenylglycine. Thereafter, according to the same procedures as described above, the (-)-isomer and the (+)-isomer can be isolated and purified in that order.

It is to be understood that the aforesaid four diastereoisomers, i.e. the (-)-N-acetyl-D-α-phenylglycine salt of the (+)-isomer, the (+)-N-acetyl-L-α-phenylglycine salt of the (-)-isomer, the (+)-N-acetyl-L-α-phenylglycine salt of the (+)-isomer, and the (-)-N-acetyl-D-α-phenylglycine salt of the (-)-isomer, are also novel compounds.

The dosage of the compounds of the present invention may vary according to the age of the patient, the severity of the disease, and administration schedule. However, they are usually administered in a dose of 1 to 50 mg for adults. The permissible daily dose should desirably be 300 mg or less.

The compounds of the present invention can be made into tablets, capsules, powders, granules, injections, syrups, suspensions, emulsions, suppositories and the like with the aid of common additives for pharmaceutical use. A variety of additives for pharmaceutical use may suitably be used according to the desired dosage form, and specific examples thereof include cellulose, lactose, sucrose, mannitol, sorbitol, starch (obtained from potatoes, corn, rice, wheat, etc.), gelatin, gum arabic, tragacanth gum, polyvinyl pyrrolidone, carboxymethyl cellulose, sodium carboxymethyl cellulose, calcium carboxymethyl cellulose, talc, magnesium stearate, polyethylene glycol, polysorbate, glycerol, cacao butter, macrogol and the like.

The central muscle relaxant activities of the compounds of the present invention were tested by animal experiments on anti-nicotine activity, anti-tremorine activity, muscle relaxant

activity and rigidity reluxing effect.

The typical compounds of the present invention used in these tests were as follows:

Table-1

| Example No. | The compounds used for the tests |
|---|---|
| 1 | 1-(4-Cyclopropylphenyl)-2-methyl-3-piperidino-1-propanone hydrochloride |
| 8 | 1-(4-Cyclopropylphenyl)-2-methyl-3-(1-pyrrolidinyl)-1-propanone hydrochloride |
| 9 | 1-(4-Cyclopropylphenyl)-2-methyl-3-(hexahydro-1*H*-azepin-1-yl)-1-propanone hydrochloride |
| 48 | 1-(4-Cyclohexylphenyl)-2-methyl-3-piperidino-1-propanone hydrochloride |
| 11 | 1-(4-Cyclopropylphenyl)-2-ethyl-3-piperidino-1-propanone hydrochloride |
| 10 | 1-(4-Cyclopropylphenyl)-2-ethyl-3-(1-pyrrolidinyl)-1-propanone hydrochloride |
| 41 | 1-(4-Cyclopentylphenyl)-2-methyl-3-piperidino-1-propanone hydrochloride |
| 5 | 1-(4-Cyclopropylphenyl)-2-isopropyl-3-piperidino-1-propanone hydrochloride |
| 37 | 2-Cyclopentyl-1-(4-cyclopropylphenyl)-3-piperidino-1-propanone hydrochloride |
| 57 | 2-Methyl-1-(3,4-methylenedioxyphenyl)-3-piperidino-1-propanone hydrochloride |
| 60 | 2-Methyl-1-(3,4-methylenedioxyphenyl)-3-(1-pyrrolidinyl)-1-propanone hydrochloride |
| 61 | 2-Methyl-1-(3,4-methylenedioxyphenyl)-3-(hexahydro-1*H*-azepin-1-yl)-1-propanone hydrochloride |
| 56 | 2-Ethyl-1-(3,4-methylenedioxyphenyl)-3-(1-pyrrolidinyl)-1-propanone hydrochloride |
| 53 | 2-Ethyl-1-(3,4-methylenedioxyphenyl)-3-piperidino-1-propanone hydrochloride |
| 66 | 1-(3,4-Methylenedioxyphenyl)-2-n-propyl-3-piperidino-1-propanone hydrochloride |
| 85 | 1-(2,3-Dihydro-5-benzofuranyl)-2-methyl-3-piperidino-1-propanone hydrochloride |
| 92 | 1-(2,3-Dihydro-5-benzofuranyl)-2-methyl-3-(1-pyrrolidinyl)-1-propanone hydrochloride |
| 93 | 1-(2,3-Dihydro-5-benzofuranyl)-2-methyl-3-(hexahydro-1*H*-azepin-1-yl)-1-propanone hydrochloride |
| 95 | 1-(2,3-Dihydro-5-benzofuranyl)-2-ethyl-3-(1-pyrrolidinyl)-1-propanone hydrochloride |

Table-1 continued

| | |
|---|---|
| 89 | 1-(2,3-Dihydro-5-benzofuranyl)-2-ethyl-3-piperidino-1-propanone hydrochloride |
| 96 | 1-(2,3-Dihydro-5-benzofuranyl)-2-ethyl-3-(hexahydro-1*H*-azepin-1-yl)-1-propanone hydrochloride |
| 98 | 1-(2,3-Dihydro-5-benzofuranyl)-2-n-propyl-3-(1-pyrrolidinyl)-1-propanone hydrochloride |
| 99 | 1-(2,3-Dihydro-5-benzofuranyl)-2-n-propyl-3-piperidino-1-propanone hydrochloride |
| 100 | 1-(2,3-Dihydro-5-benzofuranyl)-2-n-propyl-3-(hexahydro-1*H*-azepin-1-yl)-1-propanone hydrochloride |

Tolperisone Hydrochloride and Eperisone Hydrochloride were used as reference compounds.

Tolperisone HCl

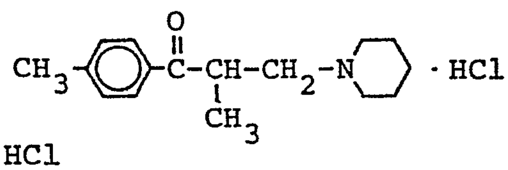

Eperisone HCl

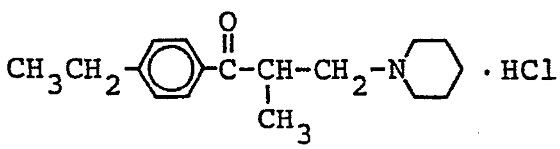

Test 1 Anti-nicotine activity

Male mice of ddY strain, weighing 28 to 33 g, were used in groups of eight or twelve. The test compounds were dissolved in distilled water and administered orally in a dose of 200 mg/kg. After an hour, nicotine was intravenously administered in a dose of 2 mg/kg. Thereafter, the occurrence of convulsive death in the mice was observed for an hour.

Inhibition of death (%)

$$= \frac{\text{Number of surviving animals}}{\text{Number of animals used}} \times 100$$

Table-2a

| Test compd. (Example No.) | Test 1 Anti-nicotine activity | | | |
|---|---|---|---|---|
| | Dose (mg/kg,po) | Number of animals used | Number of surviving animals | Inhibition of death (%) |
| Control | —— | 8 | 0 | 0 |
| 1 | 200 | 8 | 8 | 100.0 |
| 8 | 200 | 8 | 5 | 62.5 |
| 9 | 200 | 8 | 6 | 75.0 |
| 48 | 200 | 8 | 5 | 62.5 |
| 11 | 200 | 8 | 3 | 37.5 |
| 10 | 200 | 8 | 0 | 0 |
| 41 | 200 | 8 | 3 | 37.5 |
| Tolperisone HCl | 200 | 8 | 0 | 0 |
| Eperisone HCl | 200 | 8 | 4 | 50.0 |

Table-2b

| Test compd. (Example No.) | Test 1 Anti-nicotine activity | | | |
|---|---|---|---|---|
| | Dose (mg/kg,po) | Number of animals used | Number of surviving animals | Inhibition of death (%) |
| Control | —— | 12 | 0 | 0 |
| 57 | 200 | 12 | 5 | 41.7 |
| 60 | 200 | 12 | 7 | 58.3 |
| 61 | 200 | 12 | 7 | 58.3 |
| 56 | 200 | 12 | 5 | 41.7 |
| 53 | 200 | 12 | 10 | 83.3 |
| 66 | 200 | 12 | 7 | 58.3 |
| Tolperisone HCl | 200 | 12 | 0 | 0 |
| Eperisone HCl | 200 | 12 | 6 | 50.0 |

Table-2c

| Test compd. (Example No.) | Test 1 Anti-nicotine activity | | | |
|---|---|---|---|---|
| | Dose (mg/kg,po) | Number of animals used | Number of surviving animals | Inhibition of death (%) |
| Control | — | 12 | 0 | 0 |
| 85 | 200 | 12 | 8 | 66.7 |
| 92 | 200 | 12 | 9 | 75.0 |
| 93 | 200 | 12 | 7 | 58.3 |
| 95 | 200 | 12 | 2 | 16.7 |
| 89 | 200 | 12 | 8 | 66.7 |
| 96 | 200 | 12 | 5 | 41.7 |
| 98 | 200 | 12 | 0 | 0 |
| 99 | 200 | 12 | 3 | 25.0 |
| 100 | 200 | 12 | 0 | 0 |
| Tolperisone HCl | 200 | 12 | 1 | 8.3 |
| Eperisone HCl | 200 | 12 | 6 | 50.0 |

Test 2 Anti-tremorine activity

Male mice of ddY strain, weighing 28 to 32 g, were used in groups of eight or ten. The test compounds were dissolved in distilled water and administered orally in a dose of 50 mg/kg. After 30 minutes, tremorine dihydrochloride was subcutaneously administered in a dose of 20 mg/kg. Then, the occurrence of tremor was observed after the lapse of 30 and 60 minutes.

Inhibition of tremor (%)

$$= \frac{\text{Number of animals exhibiting no tremor}}{\text{Number of animals used}} \times 100$$

Table-3a

| Test compd. (Example No.) | Test 2 Anti-tremorine activity | | | | | |
|---|---|---|---|---|---|---|
| | Dose (mg/kg,po) | Number of animals used | After 30 min. | | After 60 min. | |
| | | | (a)* | (b)* | (a)* | (b)* |
| Control | 50 | 8 | 0 | 0 | 0 | 0 |
| 1 | 50 | 8 | 8 | 100 | 7 | 87.5 |
| 8 | 50 | 8 | 8 | 100 | 7 | 87.5 |
| 9 | 50 | 8 | 8 | 100 | 7 | 87.5 |
| 48 | 50 | 8 | 8 | 100 | 3 | 37.5 |
| 11 | 50 | 8 | 8 | 100 | 8 | 100.0 |
| 10 | 50 | 8 | 8 | 100 | 8 | 100.0 |
| 41 | 50 | 8 | 6 | 75 | 1 | 12.5 |
| Tolperisone HCl | 50 | 8 | 0 | 0 | 0 | 0 |
| Eperisone HCl | 50 | 8 | 7 | 87.5 | 0 | 0 |

(a)*: Number of animals exhibiting no tremor

(b)*: Inhibition of tremor (%)

Table-3b

| Test compd. (Example No.) | Test 2 Anti-tremorine activity | | | | | |
|---|---|---|---|---|---|---|
| | Dose (mg/kg,po) | Number of animals used | After 30 min. | | After 60 min. | |
| | | | (a)* | (b)* | (a)* | (b)* |
| Control | — | 10 | 0 | 0 | 0 | 0 |
| 5 | 50 | 10 | 10 | 100 | 10 | 100 |
| 37 | 50 | 10 | 8 | 80 | 8 | 80 |
| Tolperisone HCl | 50 | 10 | 0 | 0 | 0 | 0 |
| Eperisone HCl | 50 | 10 | 8 | 80 | 1 | 10 |

(a)*:Number of animals exhibiting no tremor

(b)*:Inhibition of tremor (%)

Table-3c

| Test compd. (Example No.) | Test 2 Anti-tremorine activity | | | | | |
|---|---|---|---|---|---|---|
| | Dose (mg/kg,po) | Number of animals used | After 30 min. | | After 60 min. | |
| | | | (a)* | (b)* | (a)* | (b)* |
| Control | — | 10 | 0 | 0 | 0 | 0 |
| 57 | 50 | 10 | 7 | 70 | 6 | 60 |
| 60 | 50 | 10 | 8 | 80 | 6 | 60 |
| 61 | 50 | 10 | 9 | 90 | 8 | 80 |
| 56 | 50 | 10 | 10 | 100 | 9 | 90 |
| 53 | 50 | 10 | 9 | 90 | 9 | 90 |
| 66 | 50 | 10 | 10 | 100 | 9 | 90 |
| Tolperisone HCl | 50 | 10 | 0 | 0 | 0 | 0 |
| Eperisone HCl | 50 | 10 | 8 | 80 | 1 | 10 |

(a)*: Number of animals exhibiting no tremor

(b)*: Inhibition of tremor (%)

Table-3d

| Test compd. (Example No.) | Test 2 Anti-tremorine activity | | | | | |
|---|---|---|---|---|---|---|
| | Dose (mg/kg,po) | Number of animals used | After 30 min. | | After 60 min. | |
| | | | (a)* | (b)* | (a)* | (b)* |
| Control | — | 10 | 0 | 0 | 0 | 0 |
| 85 | 50 | 10 | 10 | 100 | 10 | 100 |
| 92 | 50 | 10 | 10 | 100 | 10 | 100 |
| 93 | 50 | 10 | 10 | 100 | 10 | 100 |
| 95 | 50 | 10 | 10 | 100 | 10 | 100 |
| 89 | 50 | 10 | 10 | 100 | 10 | 100 |
| 96 | 50 | 10 | 10 | 100 | 10 | 100 |
| 98 | 50 | 10 | 10 | 100 | 9 | 90 |
| 99 | 50 | 10 | 10 | 100 | 9 | 90 |
| 100 | 50 | 10 | 10 | 100 | 10 | 100 |
| Tolperisone HCl | 50 | 10 | 0 | 0 | 0 | 0 |
| Eperisone HCl | 50 | 10 | 8 | 80 | 2 | 20 |

(a)*: Number of animals exhibiting no tremor

(b)*: Inhibition of tremor (%)

Test 3 Muscle relaxant activity

Male mice of ddY strain, weighing 30 to 37 g, were used in groups of ten. The test compounds were dissolved in physiological saline and administered intraperitoneally in a dose of 50 or 75 mg/kg. The muscle relaxant activity was evaluated according to the method of Courvoisier et al. ("Psychotropic Drugs," ed. by Garattini, S. and Ghetti, V., Elsevier Pub. Co., Amsterdam, 1957, p. 373). This method involves a traction test in which the animal is hung by its forelimbs on a horizontal metal rod (2 mm in diameter) placed 20 cm above the floor and examined as to whether it can put its hindlimb on the rod or not.

Specifically, 15 minutes after administration of a test compound, the mouse was hung on the rod by its forelimbs. When the mouse could not put its hindlimb on the rod within 5 seconds, the test compound was judged to be effective. It had been confirmed in preliminary tests that, under normal conditions, all the mice could put their hindlimb on the rod within 5 seconds.

Muscle relaxation rate (%)

$$= \frac{\text{Number of animals exhibiting muscle relaxation}}{\text{Number of animals used}} \times 100$$

Table-4a

| Test compd. (Example No.) | Test 3 Muscle relaxant activity | | |
|---|---|---|---|
| | Dose (mg/kg,ip) | Number of animals exhibiting muscle relaxation | Muscle relaxation rate (%) |
| 1 | 50 | 6 | 60 |
| 8 | 50 | 7 | 70 |
| 9 | 50 | 7 | 70 |
| Tolperisone HCl | 50 | 1 | 10 |
| Eperisone HCl | 50 | 2 | 20 |

Table-4b

| Test compd. (Example No.) | Test 3 Muscle relaxant activity | | |
|---|---|---|---|
| | Dose (mg/kg,ip) | Number of animals exhibiting muscle relaxation | Muscle relaxation rate (%) |
| 5 | 75 | 8 | 80 |
| Tolperisone HCl | 75 | 2 | 20 |
| Eperisone HCl | 75 | 4 | 40 |

Table-4c

| Test compd. (Example No.) | Test 3 Muscle relaxant activity | | |
|---|---|---|---|
| | Dose (mg/kg,ip) | Number of animals exhibiting muscle relaxation | Muscle relaxation rate (%) |
| 57 | 75 | 3 | 30 |
| 60 | 75 | 4 | 40 |
| 61 | 75 | 4 | 40 |
| 56 | 75 | 7 | 70 |
| 53 | 75 | 7 | 70 |
| 66 | 75 | 7 | 70 |
| Tolperisone HCl | 75 | 1 | 10 |
| Eperisone HCl | 75 | 4 | 40 |

Table-4d

| Test compd. (Example No.) | Test 3 Muscle relaxant activity | | |
|---|---|---|---|
| | Dose (mg/kg,ip) | Number of animals exhibiting muscle relaxation | Muscle relaxation rate (%) |
| 85 | 75 | 8 | 80 |
| 92 | 75 | 6 | 60 |
| 93 | 75 | 6 | 60 |
| 89 | 75 | 5 | 50 |
| 99 | 75 | 2 | 20 |
| Tolperisone HCl | 75 | 1 | 10 |
| Eperisone HCl | 75 | 2 | 20 |

<u>Test 4</u> Effect on decelebrate rigidity induced by transection between superior and inferior colliculi

Male rats of Wistar strain, weighing 300 to 400 g, were used. The test compounds were dissolved in physiological saline and administered intravenously in doses of 10 mg/kg and 20 mg/kg.

Rigidity preparations were made according to the method of Fukuda et al. [The Japanese Journal of Pharmacology, <u>22</u>, 457 (1972)]. Specifically, after a rat was fixed in a prone position under ether anesthesia, an incision was made in the head. The skull was removed around the occipital region to expose the brain. Then, the dura mater was cut over the cerebellum and a transection between the superior and inferior colliculi of the mid-brain was made with a spatula. More than 2 hours after operation, the rat was mounted in a supine position on a fixing stand. Thus, the effect of the test compounds was evaluated by using the occurrence of rigidity in the antigravity muscles of the limbs as an index.

Results: The test compound of Example No.1 relaxed the rigidity of the forelimbs and hindlimbs to a significant degree at a dose of 10 mg/kg, and the test compounds of Examples No.53, 57, 66, 85, 92 and 93 relaxed the rigidity similarly at a dose of 20 mg/kg.

Test 5 Acute toxicity

Male mice of ddY strain, weighing 25 to 30 g, were used in groups of five. The test compounds were dissolved in distilled water and administered orally in various doses. These mice were observed for 7 days after administration. Then, the $LD_{50}$ was calculated according to the method of Behrens and Kärbar.

Table-5a

| Test compd. (Example No.) | Test 5 Acute toxicity $LD_{50}$ (mg/kg,po) |
|---|---|
| 1 | 535 |
| 8 | 505 |
| 9 | 655 |
| Tolperisone HCl | 625 |
| Eperisone HCl | 595 |

Table-5b

| Test compd. (Example No.) | Test 5 Acute toxicity $LD_{50}$ (mg/kg,po) |
|---|---|
| 57 | 625 |
| 53 | 565 |
| 66 | 625 |
| Tolperisone HCl | 655 |
| Eperisone HCl | 475 |

Table-5c

| Test compd. (Example No.) | Test 5 Acute toxicity $LD_{50}$ (mg/kg,po) |
|---|---|
| 85 | 625 |
| 92 | 685 |
| 93 | 685 |
| Tolperisone HCl | 595 |
| Eperisone HCl | 565 |

The pharmaceutical composition of the present invention is further illustrated with reference to several specific examples. However, these examples are not intended to limit the scope of the present invention.

Tablets

| | |
|---|---|
| Compound of Example No.1 .................... | 100 g |
| Crystalline cellulose ....................... | 150 g |
| Corn starch ................................. | 142 g |
| Lactose ..................................... | 300 g |
| Calcium carboxymethyl cellulose ............. | 5 g |
| Magnesium stearate .......................... | 3 g |
| Total | 700 g |

The above ingredients are blended well by means of an ordinary mixer. Immediately after that, or after being formed into fine granules or granules, the blend is compressed to form a total of 5,000 tablets. Each tablet weighs 140 mg and contains 20 mg of Compound of Example No.1 used as the active ingredient. If desired, these tablets may be made into sugar-coated or film-coated tablets according to conventional procedure.

Capsules

| | |
|---|---|
| Compound of Example No.53 ................... | 50 g |
| Powdered lactose ............................ | 146 g |
| Talc ........................................ | 4 g |
| Total | 200 g |

The above ingredients are blended well and filled into a total of 1,000 capsules by means of a capsule filling machine. Each of the capsules so produced contains 50 mg of Compound of Example No.53 used as the active ingredient.

Injections

10 g of Compound of Example No.85 is dissolved in 1,000 ml of distilled water for injections. Using a dispensing machine, 1 ml each of the solution is filled into a total of 1,000 ampules. Each of the ampules so produced contains 10 mg of Compound of Example No.85 used as the active ingredient. Alternatively, a solution having the same concentration may be

filled into vials with any desired capacity.

The process for preparing the compounds of the present invention is more specifically illustrated by the following examples.

For use as intermediates in these examples, several 4'-cycloalkylphenone compounds, 3',4'-methylenedioxyphenone compounds and 2,3-dihydro-5-benzofuranylketone compounds of formula (IV) were prepared according to the procedures described in the following reference examples.

Reference Example 1

4'-Cyclopropylpropiophenone

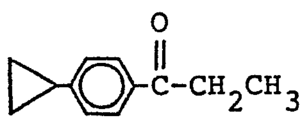

19.6 g of propionyl chloride and 28.6 g of aluminum chloride were dissolved in 200 ml of chloroform. While the resulting solution was being kept at a temperature of -55 to -50°C, 25.0 g of cyclopropylbenzene was added dropwise thereto over a period of an hour. After completion of the addition, the stirring was continued for an additional 10 minutes. Thereafter, the reaction mixture was poured into 40 ml of concentrated hydrochloric acid containing 300 g of ice blocks, followed by vigorous stirring. The chloroform layer was isolated, washed with water and then dried over anhydrous sodium sulfate. The solvent was distilled off under reduced pressure to obtain an oily residue, which was distilled at 125 -128°C under a reduced pressure of 5 mmHg to obtain 18.8 g (51%) of 4'-cyclopropyl-propiophenone.

NMR($CDCl_3$) δ ppm: 0.63 - 1.16(4H,m), 1.18(3H,t), 1.67 - 2.20 (1H,m), 2.92(2H,q), 7.05(2H,d), 7.78(2H,d)

Reference Example 2

4'-Cyclopentylpropiophenone

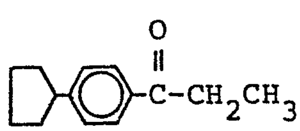

21.3 g of aluminum chloride was suspended in 95 ml of carbon tetrachloride. Under cooling with ice, 14.8 g of propionyl chloride was added thereto and the mixture was stirred for 15 minutes. While the resulting solution was being kept at a reaction temperature of 5°C or below, 20.0 g of cyclopentylbenzene was added dropwise thereto over a period of 3 hours. After completion of the addition, the stirring was continued for an additional hour. Thereafter, the reaction mixture was poured into 160 ml of concentrated hydrochloric acid containing 200 g of ice blocks, followed by vigorous stirring. The carbon tetrachloride layer was isolated, washed with water and then dried over anhydrous sodium sulfate. The solvent was distilled off under reduced pressure to obtain an oily residue, which was distilled at 148 - 150°C under a reduced pressure of 5 mmHg to obtain 23.0 g (83%) of 4'-cyclopentylpropiophenone.

NMR($CDCl_3$) δ ppm: 0.90 - 2.30(8H,m), 1.18(3H,t), 2.50 - 3.33 (1H,m), 2.93(2H,q), 7.27(2H,d), 7.80(2H,d)

Reference Example 3

4'-Cyclopropyl-3-methylbutyrophenone

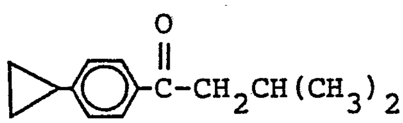

31.1 g of aluminum chloride was suspended in 200 ml of methylene chloride. Under cooling with ice, 27.7 g of isovaleryl chloride was added thereto and the mixture was stirred for 15 minutes. While the resulting solution was being kept at a temperature of -65 to -70°C, 27.2 g of cyclopropylbenzene was added dropwise thereto over a period of 25 minutes. After completion of the addition, the stirring was continued for an additional 35 minutes. Thereafter, the reaction mixture was poured into 45 ml of concentrated hydrochloric acid containing 300 g of ice blocks, followed by vigorous stirring. The resulting methylene chloride layer was isolated, washed with water and then dried over anhydrous sodium sulfate. The solvent was distilled off under reduced pressure to obtain an oily residue, which was distilled under reduced pressure to obtain 27.9 g (60%) of 4'-cyclopropyl-3-methylbutyrophenone, B.P. 144.0 - 146.0°C at 4 mmHg.

NMR($CDCl_3$) δ ppm: 0.53 - 1.26(4H,m), 0.98(6H,d), 1.67 -

2.58(2H,m), 2,78(2H,d), 7.03(2H,d), 7.77(2H,d)

Reference Example 4

3',4'-Methylenedioxybutyrophenone

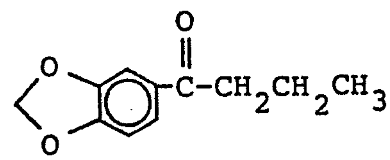

28.1 g of 1,2-methylenedioxybenzene and 26.6 g of butyryl chloride were dissolved in 200 ml of methylene chloride. While the resulting solution was being kept at a temperature of 10°C or below, 75.0 g of tin(IV) chloride was added dropwise thereto over a period of 30 minutes. After completion of the addition, the stirring was continued for an additional 10 minutes. Thereafter, the reaction mixture was poured into 150 ml of concentrated hydrochloric acid containing 200 g of ice blocks, followed by vigorous stirring. The methylene chloride layer was isolated, washed with water and then dried over anhydrous sodium sulfate. The solvent was distilled off under reduced pressure to obtain a residue, which was recrystallized from hexane to obtain 33.1 g (75%) of 3',4'-methylenedioxybutyrophenone in the form of white crystals, M.P. 41.0 - 43.0°C.

NMR($CDCl_3$) δ ppm: 1.00(3H,t), 1.73(2H,sextet), 2.83(2H,t), 5.97(2H,s), 6.76(1H,d), 7.38(1H,d), 7.50(1H,dd)

Reference Example 5

5-Propionyl-2,3-dihydrobenzofuran

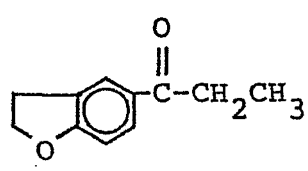

18.4 g of 2,3-dihydrobenzofuran and 15.3 g of propionyl chloride were dissolved in 120 ml of methylene chloride. While the resulting solution was being kept at a temperature of 10°C or below, 50.0 g of tin(IV) chloride was added dropwise thereto over a period of 30 minutes. After completion of the addition, the stirring was continued for an additional 10 minutes. Thereafter, the reaction mixture was poured into 100 ml of concentrated hydrochloric acid containing 200 g of ice blocks, followed by vigorous stirring. The methylene chloride layer was isolated, washed with water and then dried over anhydrous

sodium sulfate. The solvent was distilled off under reduced pressure to obtain a residue, which was recrystallized from hexane to obtain 20.5 g (76%) of 5-propionyl-2,3-dihydrobenzofuran in the form of white crystals, M.P. 60.0 - 61.0°C.

NMR($CDCl_3$) δ ppm: 1.18(3H,t), 2.88(2H,q), 3.15(2H,t), 4.58(2H,t), 6.69(1H,d), 7.58 - 7.88(2H,m)

By the application of the similar procedures as described in Reference Examples 1 - 5, 4'-cycloalkylphenone compounds, 3',4'-methylenedioxyphenone compounds or 2,3-dihydro-5-benzofuranylketone compounds of Reference Examples 6 - 37 below were obtained.

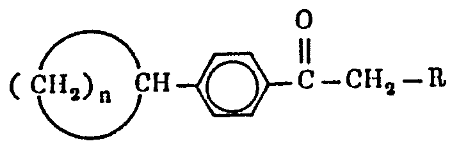

Table -6

| Reference Example No. | n | R | Boiling point (b.p.) or Melting point (m.p.) | NMR($CDCl_3$) δ ppm |
|---|---|---|---|---|
| 6 | 2 | $-CH_2CH_3$ | b.p. 120-125℃/3mmHg | 0.58-1.22(4H, m), 1.00(3H, t), 1.45-2.22(3H, m), 2.88(2H, t), 7.05(2H, d), 7.80(2H, d) |
| 7 | 2 | $-CH_2CH_2CH_3$ | 〃 120℃/5mmHg | 0.45-2.15(12H, m), 2.88(2H, t), 7.00(2H, d), 7.78(2H, d) |
| 8 | 2 | $-CH_2(CH_2)_2CH_3$ | 〃 145-158℃/4mmHg | 0.58-2.18(14H, m), 2.90(2H, t), 7.07(2H, d), 7.82(2H, d) |
| 9 | 2 | $-CH_2(CH_2)_3CH_3$ | 〃 140℃/3mmHg | 0.57-2.20(16H, m), 2.88(2H, t), 7.03(2H, d), 7.80(2H, d) |
| 10 | 2 | $-CH_2(CH_2)_4CH_3$ | m.p. 41.5-42.5℃ | 0.57-2.70(18H, m), 2.88(2H, t), 7.03(2H, d), 7.78(2H, d) |
| 11 | 4 | $-CH_2CH_3$ | b.p. 158-160℃/5mmHg | 0.67-2.40(10H, m), 0.98(3H, t), 2.50-3.43(1H, t), 2.90(2H, t), 7.28(2H, d), 7.87(2H, d) |
| 12 | 5 | $-CH_3$ | m.p. 50.0-51.0℃ | 0.62-3.22(11H, m), 1.18(3H, t), 2.95(2H, q), 7.20(2H, d), 7.83(2H, d) |
| 13 | 5 | $-CH_2CH_3$ | b.p. 171-175℃/6mmHg | 0.67-3.10(13H, m), 0.98(3H, t), 2.88(2H, t), 7.24(2H, d), 7.87(2H, d) |

Table-6 continued

| | | | | |
|---|---|---|---|---|
| 14 | 2 | $-CH_2CH(CH_3)_2$ | b.p. 155.0-160.0° C/5mmHg | 0.42-2.30(8H, m), 0.93(6H, d), 2.87(2H, t), 7.03(2H, d), 7.78(2H, d) |
| 15 | 2 | 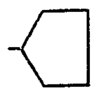 | b.p. 175.0-180.0°C/4mmHg | 0.53-2.67(14H, m), 2.83(2H, d), 6.96(2H, d), 7.70(2H, d) |
| 16 | 2 | $-CH_2-$ 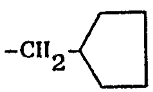 | b.p. 180.0-200.0°C/4mmHg | 0.57-2.50(16H, m), 2.87(2H, t), 7.03(2H, d), 7.80(2H, d) |
| 17 | 2 | 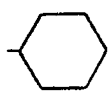 | b.p. 147.0-148.0°C/ 1.5mmHg | 0.57-2.50(16H, m), 2.73(2H, d), 7.04(2H, d), 7.81(2H, d) |

Table-7 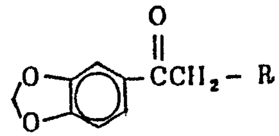

| Reference Example No. | $R_1$ | Boiling point (b.p.) or Melting point (m.p.) | NMR($CDCl_3$) δ ppm |
|---|---|---|---|
| 18 | $-CH_3$ | m.p. 37.0 - 39.0℃ | 1.17(3H, t), 2.88(2H, q), 5.97(2H, s), 6.75(1H, d), 7.34(1H, d), 7.47(1H, dd) |
| 19 | $-n-C_3H_7$ | b.p. 130℃/5mmHg | 0.40 - 2.07(7H, m), 2.85(2H, t), 5.97(2H, s), 6.76(1H, d), 7.37(1H, d), 7.50(1H, dd) |
| 20 | $-iso-C_3H_7$ | b.p. 141 - 144℃/4mmHg | 0.98(6H, d), 1.90 - 2.57(1H, m), 2.73(2H, d), 5.95(2H, s), 6.75(1H, d), 7.37(1H, d), 7.46(1H, dd) |
| 21 | $-n-C_4H_9$ | m.p. 40.0 - 41.0℃ | 0.50 - 2.07(9H, m), 2.86(2H, t), 5.97(2H, s), 6.78(1H, d), 7.37(1H, d), 7.51(1H, dd) |
| 22 | $-iso-C_4H_9$ | oil | 0.92(6H, d), 1.23 - 1.90(3H, m), 2.80(2H, t), 5.93(2H, s), 6.70(1H, d), 7.30(1H, d), 7.45(1H, dd) |
| 23 | $-n-C_5H_{11}$ | m.p. 44.0 - 45.0℃ | 0.65 - 2.12(11H, m), 2.84(2H, t), 5.98(2H, s), 6.78(1H, d), 7.36(1H, d), 7.50(1H, dd) |
| 24 | $-n-C_6H_{13}$ | m.p. 31.5 - 32.5℃ | 0.60 - 2.10(13H, m), 2.88(2H, t), 6.00(2H, s), 6.80(1H, d), 7.38(1H, d), 7.52(1H, dd) |
| 25 | 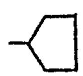 | m.p. 48.0 - 49.0℃ | 0.65 - 2.62(9H, m), 2.87(2H, d), 5.97(2H, s), 6.70(1H, d), 7.32(1H, d), 7.45(1H, dd) |

Table-7 continued

| | | | |
|---|---|---|---|
| 26 | $-CH_2$ 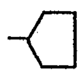 | m.p. 73℃ | 0.63 - 2.30 (11H, m), 2.85 (2H, t), 5.95 (2H, s), 6.73 (1H, d), 7.32 (1H, d), 7.45 (1H, dd) |
| 27 | 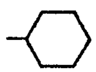 | b.p. 147.0 - 148.0℃/1.5mmHg | 0.57 - 2.43 (11H, m), 2.73 (2H, d), 6.00 (2H, s), 6.77 (1H, d), 7.40 (1H, d), 7.49 (1H, dd) |

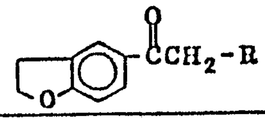

Table - 8

| Reference Example No. | R | Boiling point(b.p.) or Melting point(m.p.) | NMR($CDCl_3$)$\delta$ ppm |
|---|---|---|---|
| 28 | $-C_2H_5$ | m.p. 42.0℃ | 0.95(3H,t), 1.68(2H,sextet), 2.75(2H,t), 3.12(2H,t), 4.53(2H,t), 6.62(1H,d), 7.42-7.85(2H,m) |
| 29 | $-n-C_3H_7$ | b.p. 134.0-140.0℃/1mmHg | 0.67-2.00(7H,m), 2.83(2H,t), 3.17(2H,t), 4.58(2H,t), 6.68(1H,d), 7.57-7.90(2H,m) |
| 30 | $-iso-C_3H_7$ | m.p. 72.5-73.5℃ | 0.99(6H,d), 1.77-2.67(1H,m), 2.73(2H,d), 3.17(2H,t), 4.60(2H,t), 6.73(1H,d), 7.63-7.97(2H,m) |
| 31 | $-n-C_4H_9$ | b.p. 144.0-146.0℃/4mmHg | 0.70-2.13(9H,m), 2.87(2H,t), 3.20(2H,t), 4.62(2H,t), 6.74(1H,d), 7.67-8.03(2H,m) |
| 32 | $-iso-C_4H_9$ | m.p. 36.0℃ | 0.92(6H,d), 1.17-1.83(3H,m), 2.77(2H,t), 3.13(2H,t), 4.53(2H,t), 6.63(1H,d), 7.53-7.87(2H,m) |
| 33 | $-n-C_5H_{11}$ | oil | 0.63-2.03(11H,m), 2.85(2H,t), 3.18(2H,t), 4.60(2H,t), 6.72(1H,d), 7.63-8.00(2H,m) |
| 34 | $-n-C_6H_{13}$ | m.p. 39.0℃ | 0.53-2.03(13H,m), 2.88(2H,t), 3.22(2H,t), 4.62(2H,t), 6.73(1H,d), 7.63-7.97(2H,m) |
| 35 | 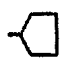 | m.p. 84.0℃ | 0.65-2.65(9H,m), 2.88(2H,d), 3.22(2H,t), 4.62(2H,t), 6.74(1H,d), 7.63-7.95(2H,m) |
| 36 | $-CH_2-$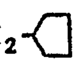 | m.p. 62.0℃ | 0.63-2.20(11H,m), 2.88(2H,t), 3.20(2H,t), 4.60(2H,t), 6.73(1H,d), 7.63-7.97(2H,m) |
| 37 | 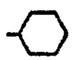 | m.p. 87.0-88.0℃ | 0.38-2.42(11H,m), 2.70(2H,d), 3.17(2H,t), 4.58(2H,t), 6.71(1H,d), 7.60-7.95(2H,m) |

Example 1

1-(4-Cyclopropylphenyl)-2-methyl-3-piperidino-1-propanone hydrochloride

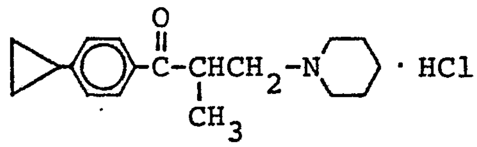

2.94 g of the 4'-cyclopropylpropiophenone prepared in Reference Example 1, 0.66 g of paraformaldehyde, 2.46 g of piperidine hydrochloride and one drop of concentrated hydrochloric acid were added to 1.5 ml of isopropanol and the resulting reaction mixture was heated under reflux for 2.5 hours. After the reaction mixture was allowed to cool, 50 ml of chloroform was added thereto and the resulting mixture was washed three times with 30-ml portions of a saturated aqueous solution of sodium chloride. After the chloroform layer was dried over anhydrous sodium sulfate, the solvent was distilled off under reduced pressure to obtain a residue, which was recrystallized from a solvent mixture of isopropanol and ethyl ether to obtain 4.20 g (81%) of 1-(4-cyclopropylphenyl)-2-methyl-3-piperidino-1-propanone hydrochloride in the form of white crystals, M.P. 174.5 - 175.5°C (dec.).

IR(KBr)$cm^{-1}$: 2950, 2930, 2870, 2800 - 2150, 1670, 1600

NMR($CDCl_3$) δ ppm: 0.66 - 4.19(17H,m), 1.32(3H,d), 4.19 - 4.92(1H,m), 7.12(2H,d), 7.97(2H,d), 11.96(1H,br)

Elemental analysis for $C_{18}H_{25}NO \cdot HCl$

| | | C | H | N |
|---|---|---|---|---|
| Calcd. | % | 70.22 | 8.51 | 4.55 |
| Found | % | 69.96 | 8.58 | 4.45 |

Example 2

1-(4-Cyclopropylphenyl)-2-methyl-3-piperidino-1-propanone

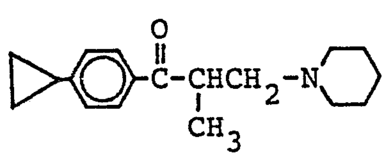

2.00 g of the 1-(4-cyclopropylphenyl)-2-methyl-3-piperidino-1-propanone hydrochloride obtained in Example 1 was dissolved in 30 ml of water and the resulting solution was alkalified with a 20 % aqueous solution of sodium hydroxide. The oily material so separated was extracted with ethyl ether and washed three times with a saturated aqueous solution of sodium chloride. The ether layer was isolated, dried over anhydrous sodium sulfate and then distilled under reduced pressure to remove the solvent. The resulting oily residue was purified by silica gel column chromatography [using an eluent composed of chloroform and ethanol (20:1)] to obtain 1.68 g (95%) of 1-(4-cyclopropylphenyl)-2-methyl-3-piperidino-1-propanone in the form of an oily material.

IR(Neat)$cm^{-1}$: 3070, 3000, 2930, 2850, 2830, 1675, 1600

NMR($CDCl_3$) δ ppm: 0.57 - 3.00(17H,m), 1.13(3H,d), 3.67(1H, sextet), 7.08(2H,d), 7.83(2H,d)

Example 3

1-(4-Cyclopropylphenyl)-2-methyl-3-piperidino-1-propanone nitrate

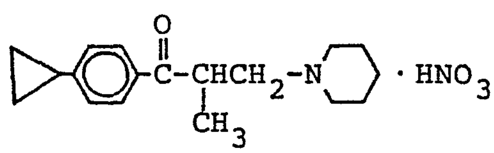

1.00 g of the 1-(4-cyclopropylphenyl)-2-methyl-3-piperidino-1-propanone obtained in Example 2 was dissolved in 10 ml of methanol and the resulting solution was acidified with 30 % nitric acid. Then, the solvent was distilled off under reduced pressure to obtain a residue, to which ethyl ether was added. The resulting mixture was stirred vigorously. The crystals so precipitated were collected by filtration and recrystallized from a solvent mixture of isopropanol and ethyl ether to obtain 0.99 g (80 %) of 1-(4-cyclopropylphenyl)-2-methyl-3-piperidino-1-propanone nitrate, M.P. 130 - 132°C (dec.).

IR(KBr)$cm^{-1}$: 3000, 2940, 2860, 2800– 2200, 1670, 1600, 1385

NMR($CDCl_3$) δ ppm: 0.62 - 4.65(18H,m), 1.28(3H,d), 7.14(2H, d), 7.90(2H,d), 10.70(1H,br)

Elemental analysis for $C_{18}H_{25}NO{\cdot}HNO_3$

| | | C | H | N |
|---|---|---|---|---|
| Calcd. | % | 64.65 | 7.84 | 8.38 |
| Found | % | 64.57 | 7.90 | 8.44 |

Example 4

1-(4-Cyclopropylphenyl)-2-methyl-3-piperidino-1-propanone succinate

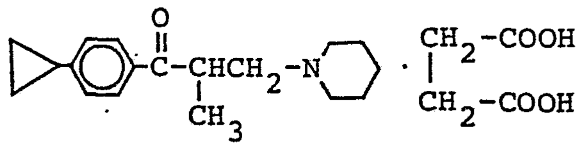

1.00 g of the 1-(4-cyclopropylphenyl)-2-methyl-3-piperidino-1-propanone obtained in Example 2 was dissolved in 10 ml of methanol. To the resulting solution was added a solution of 0.44 g of succinic acid in 5 ml of methanol. After vigorous stirring, the solvent was distilled off under reduced pressure to obtain a residue, to which a small amount of acetone was added. This residue crystallized upon standing. The crystals so formed were collected by filtration and recrystallized from a solvent mixture of acetone and ethyl ether to obtain 1.01 g (70 %) of 1-(4-cyclopropylphenyl)-2-methyl-3-piperidino-1-propanone succinate, M.P. 83 - 85°C.

IR(KBr)$cm^{-1}$: 2960, 2940, 2860, 2800 - 2200, 1710, 1670, 1640, 1600

NMR($CDCl_3$) δ ppm: 0.47 - 2.20(11H,m), 1.23(3H,d), 2.48(4H, s), 2.67 - 4.50(7H,m), 7.13(1H,d), 7.88(2H,d), 13.48(2H,br)

Elemental analysis for $C_{18}H_{25}NO \cdot C_4H_6O_4$

| | | C | H | N |
|---|---|---|---|---|
| Calcd. | % | 67.84 | 8.02 | 3.60 |
| Found | % | 67.99 | 8.10 | 3.67 |

Example 5

1-(4-Cyclopropylphenyl)-2-isopropyl-3-piperidino-1-propanone hydrochloride

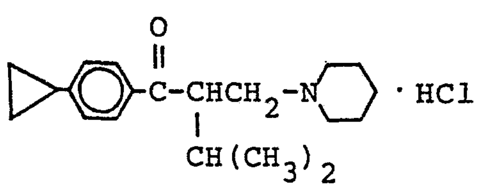

6.0 g of the 4'-cyclopropyl-3-methylbutyrophenone prepared in Reference Example 3, 1.2 g of paraformaldehyde, 4.4 g of piperidine hydrochloride and 0.2 ml of concentrated hydrochloric acid were added to 4 ml of isopropanol and the resulting reaction mixture was heated under reflux for 7 hours. After the reaction mixture was allowed to cool, it was dissolved

in 50 ml of water and the resulting solution was washed twice with ethyl ether. Then a saturated aqueous solution of sodium chloride was added to the aqueous layer, which was extracted with chloroform. This procedure was repeated twice more. The chloroform layer was washed twice with a saturated aqueous solution of sodium chloride and then dried over anhydrous sodium sulfate. The solvent was distilled off under reduced pressure to obtain a residue, which was recrystallized from a solvent mixture of isopropanol and tetrahydrofuran to obtain 5.3 g (53 %) of 1-(4-cyclopropylphenyl)-2-isopropyl-3-piperidino-1-propanone hydrochloride in the form of white crystals, M.P. 180.0 - 180.5°C.

IR(KBr)$cm^{-1}$: 2970, 2950, 2910, 2890, 2800 - 2000, 1680, 1600

NMR($CDCl_3$) δ ppm: 0.53 - 4.13(18H,m), 0.80(3H,d), 1.16(3H,d), 4.27 - 4.77(1H,m), 7.13(2H,d), 7.97(2H,d), 12.03(1H,br)

Elemental analysis for $C_{20}H_{29}NO{\cdot}HCl$

| | | C | H | N |
|---|---|---|---|---|
| Cacld. | % | 71.51 | 9.00 | 4.17 |
| Found | % | 71.68 | 8.90 | 4.22 |

Example 6

(+)-1-(4-Cyclopropylphenyl)-2-methyl-3-piperidino-1-propanone hydrochloride

(+)-isomer 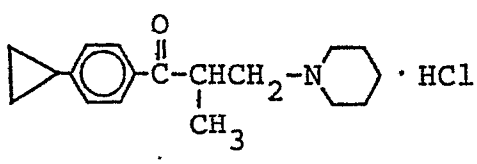

(a) (+)-1-(4-Cyclopropylphenyl)-2-methyl-3-piperidino-1-propanone (-)-N-acetyl-D-α-phenylglycinate

22.3 g of 1-(4-cyclopropylphenyl)-2-methyl-3-piperidino-1-propanone hydrochloride prepared according to the procedure of Example 1 was dissolved in 300 ml of water and the resulting solution was alkalified with a 10 % aqueous solution of sodium hydroxide, followed by vigorous stirring. The oily material so separated was extracted three times with 100-ml portions of benzene. The benzene layer was washed with water, dried over anhydrous sodium sulfate and then distilled under reduced pressure to remove the solvent. The resulting oily residue was dissolved in 100 ml of acetone, and 14.0 g of (-)-N-acetyl-

D-α-phenylglycine was added thereto and dissolved therein by the application of heat. After the sulution was allowed to cool, the solvent was distilled off under reduced pressure and the resulting oily residue was crystallized with ethyl acetate. The crystals so formed were recrystallized three times from ethyl acetate to obtain 8.5 g of (+)-1-(4-cyclopropylphenyl)-2-methyl-3-piperidino-1-propanone (-)-N-acetyl-D-α-phenylglycinate, $[\alpha]_D^{23}$ -45.3°(C=1, methanol), M.P. 104.0 - 105.0°C.

IR(KBr)$cm^{-1}$: 3260, 3080, 2960, 2940, 2800 - 2000, 1670, 1610, 1550

NMR($CDCl_3$) δ ppm: 0.58 - 2.32(11H,m), 1.03(3H,d), 2.02(3H, s), 2.42 - 4.25(7H,m), 5.33(1H,d), 6.92 - 7.98(10H,m), 13.75 (1H,br)

Elemental analysis for $C_{28}H_{36}N_2O_4$

| | | C | H | N |
|---|---|---|---|---|
| Calcd. | % | 72.39 | 7.81 | 6.03 |
| Found | % | 72.43 | 7.77 | 6.16 |

(b) (+)-1-(4-Cyclopropylphenyl)-2-methyl-3-piperidino-1-propanone hydrochloride

5.0 g of the (+)-1-(4-cyclopropylphenyl)-2-methyl-3-piperidino-1-propanone (-)-N-acetyl-D-α-phenylglycinate obtained in (a) was dissolved in 50 ml of water and the resulting solution was alkalified with a 10 % aqueous solution of sodium hydroxide. The oily material so separated was extracted three times with 50-ml portions of benzene. The benzene layer was washed with water, dried over anhydrous sodium sulfate and then concentrated under reduced pressure to a volume of about 50 ml. This solution was acidified by the addition of benzene saturated with hydrogen chloride and then distilled under reduced pressure to remove the solvent. The resulting solid residue was recrystallized from tetrahydrofuran to obtain 2.2 g of (+)-1-(4-cyclopropylphenyl)-2-methyl-3-piperidino-1-propanone hydrochloride, $[\alpha]_D^{23}$ +41.2°(C=1, methanol), M.P. 170.5 - 171.5°C.

IR(KBr)$cm^{-1}$: 2940, 2860, 2800 - 2200, 1675, 1600

NMR($CDCl_3$) δ ppm: 0.57 - 4.12(17H,m), 1.28(3H,d), 4.22 - 4.92(1H,m), 7.09(2H,d), 7.91(2H,d), 12.04(1H,br)

Elemental analysis for $C_{18}H_{25}NO\cdot HCl$

| | | C | H | N |
|---|---|---|---|---|
| Calcd. | % | 70.22 | 8.51 | 4.55 |
| Found | % | 70.16 | 8.49 | 4.61 |

Example 7

(-)-1-(4-Cyclopropylphenyl)-2-methyl-3-piperidino-1-propanone hydrochloride

(-)-isomer

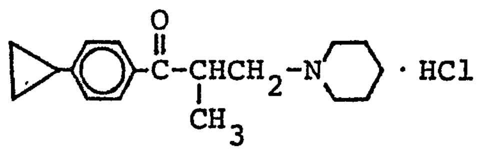

(a) (-)-1-(4-Cyclopropylphenyl)-2-methy-3-piperidino-1-propanone (+)-N-acetyl-L-α-phenylglycinate

The crystallization and recrystallization mother liquors obtained in Example 6(a) were combined and concentrated under reduced pressure. The resulting residue was dissolved in 300 ml of water and this solution was alkalified with a 10 % aqueous solution of sodium hydroxide. The oily material so separated was extracted three times with 150-ml portions of benzene. The benzene layer was washed with water, dried over anhydrous sodium sulfate and then distilled under reduced pressure to remove the solvent. Thus, there was obtained 11.4 g of an oily material containing a large amount of the (-)-isomer. This oily material was dissolved in 50 ml of acetone, and 8.1 g of (+)-N-acetyl-L-α-phenylglycine was added thereto and dissolved therein by the application of heat. After the solution was allowed to cool, the solvent was distilled off under reduced pressure and the resulting solid residue was recrystallized three times from ethyl acetate to obtain 5.9 g of (-)-1-(4-cyclopropylphenyl)-2-methyl-3-piperidino-1-propanone (+)-N-acetyl-L-α-phenylglycinate, $[\alpha]_D^{23}$ +46.8°(C=1, methanol), M.P. 102.5 - 103.5°C.

IR(KBr)$cm^{-1}$: 3250, 3060, 2950, 2930, 2800 - 2000, 1665, 1600, 1540

NMR($CDCl_3$) δ ppm: 0.58 - 2.25(11H,m), 1.02(3H,d), 2.02(3H, s), 2.35 - 4.22(7H,m), 5.27(1H,d), 6.88 - 7.92(10H,m), 13.55 (1H,br)

Elemental analysis for $C_{28}H_{36}N_2O_4$

| | | C | H | N |
|---|---|---|---|---|
| Calcd. | % | 72.39 | 7.81 | 6.03 |
| Found | % | 72.44 | 7.86 | 5.90 |

(b) (-)-1-(4-Cyclopropylphenyl)-2-methyl-3-piperidino-1-propanone hydrochloride

2.0 g of the (-)-1-(4-cyclopropylphenyl)-2-methyl-3-piperidino-1-propanone (+)-N-acetyl-L-α-phenylglycinate obtained in (a) was dissolved in 30 ml of water and the resulting solution was alkalified with a 10 % aqueous solution of sodium hydroxide. The oily material so separated was extracted three times with 20-ml portions of benzene. The benzene layer was washed with water, dried over anhydrous sodium sulfate and then concentrated under reduced pressure to a volume of about 30 ml. This solution was acidified by the addition of benzene saturated with hydrogen chloride and then distilled under reduced pressure to remove the solvent. The resulting solid residue was recrystallized from tetrahydrofuran to obtain 1.0 g (-)-1-(4-cyclopropylphenyl)-2-methyl-3-piperidino-1-propanone hydrochloride, $[\alpha]_D^{23}$ -41.5° (C=1, methanol), M.P. 170.5 - 171.5°C.

IR(KBr)$cm^{-1}$: 2940, 2860, 2800 - 2200, 1675, 1600

NMR($CDCl_3$) δ ppm: 0.57 - 4.13(17H,m), 1.29(3H,d), 4.20 - 4.93(1H,m), 7.13(2H,d), 7.93(2H,d), 12.05(1H,br)

Elemental analysis for $C_{18}H_{25}NO \cdot HCl$

| | | C | H | N |
|---|---|---|---|---|
| Calcd. | % | 70.22 | 8.51 | 4.55 |
| Found | % | 70.29 | 8.43 | 4.59 |

By the application of the similar procedure as described in Example 1, the compounds of Examples 8 - 40 below were obtained.

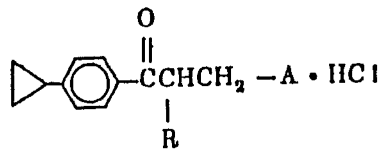

Table - 9

| Example No. | R | A | Melting point ℃ Recrystn. solvent | IR(KBr) $cm^{-1}$ | NMR($CDCl_3$) δ ppm | Molecular formula | Elemental analysis upper row Calcd. lower row Found | | |
|---|---|---|---|---|---|---|---|---|---|
| | | | | | | | C % | H % | N % |
| 8 | $-CH_3$ | 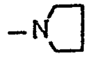 | 145.8 - 146.5<br>isopropanol-ethylether | 2980, 2930, 2880, 2700-2100, 1670, 1600 | 0.62 - 4.08 (15H, m), 1.29 (3H, d), 4.25 - 4.85 (1H, m), 7.13 (2H, d), 7.97 (2H, d), 12.33 (1H, br) | $C_{17}H_{23}NO \cdot HCl$ | 69.49<br>69.52 | 8.23<br>8.28 | 4.77<br>4.68 |
| 9 | 〃 | 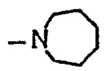 | 173.0 - 174.0 (dec.)<br>isopropanol | 2960, 2940, 2870, 2800-2200, 1670, 1600 | 0.68 - 4.25 (19H, m), 1.33 (3H, d), 4.28 - 4.88 (1H, m), 7.13 (2H, d), 7.97 (2H, d), 12.27 (1H, br) | $C_{19}H_{27}NO \cdot HCl$ | 70.90<br>70.88 | 8.77<br>8.69 | 4.35<br>4.28 |
| 10 | $-C_2H_5$ | 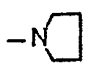 | 147.0 - 149.0<br>tetrahydrofuran | 2950, 2880, 2800-2200, 1675, 1605 | 0.70 - 4.13 (17H, m), 0.92 (3H, t), 4.33 - 4.83 (1H, m), 7.18 (2H, d), 8.03 (2H, d), 12.43 (1H, br) | $C_{18}H_{25}NO \cdot HCl$ | 70.22<br>70.19 | 8.51<br>8.60 | 4.55<br>4.39 |
| 11 | 〃 | 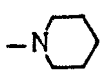 | 152.0 - 155.0<br>tetrahydrofuran-ethylether | 2950, 2920, 2860, 2800-2200, 1660, 1600 | 0.67 - 4.13 (19H, m), 0.92 (3H, t), 4.30 - 4.80 (1H, m), 7.15 (2H, d), 7.99 (2H, d), 12.07 (1H, br) | $C_{19}H_{27}NO \cdot HCl$ | 70.90<br>70.75 | 8.77<br>8.73 | 4.35<br>4.40 |
| 12 | 〃 | 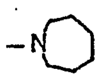 | 160.0 - 161.0<br>acetone | 2960, 2930, 2850, 2800-2200, 1660, 1600 | 0.67 - 4.27 (21H, m), 0.92 (3H, t), 4.30 - 4.83 (1H, m), 7.17 (2H, d), 8.00 (2H, d), 12.10 (1H, br) | $C_{20}H_{29}NO \cdot HCl$ | 71.51<br>71.46 | 9.00<br>8.93 | 4.17<br>4.29 |

Table-9 continued

| | | | | | | | | | |
|---|---|---|---|---|---|---|---|---|---|
| 13 | $-n-C_3H_7$ | 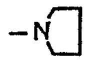 | 170.0 - 172.0 (dec.) tetrahydrofuran | 2950, 2920, 2860, 2750- 2200, 1660, 1600 | 0.67 - 4.13 (22H, m), 4.33 - 4.87 (1H, m), 7.17 (2H, d), 8.04 (2H, d), 12.47 (1H, br) | $C_{10}H_{27}NO \cdot HCl$ | 70.90<br>71.01 | 8.77<br>8.63 | 4.35<br>4.33 |
| 14 | " | 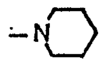 | 166.0 - 168.0 (dec.) " | 2960, 2930, 2860, 2800- 2300, 1665, 1595 | 0.67 - 4.10 (24H, m), 4.30 - 4.83 (1H, m), 7.13 (2H, d), 7.97 (2H, d), 12.20 (1H, br) | $C_{20}H_{29}NO \cdot HCl$ | 71.51<br>71.43 | 9.00<br>9.02 | 4.17<br>4.20 |
| 15 | " | 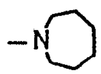 | 165.0 - 166.0 (dec.) " | 2960, 2930, 2870, 2800- 2300, 1665, 1595 | 0.68 - 4.28 (26H, m), 4.32 - 4.88 (1H, m), 7.15 (2H, d), 8.00 (2H, d), 12.18 (1H, br) | $C_{21}H_{31}NO \cdot HCl$ | 72.08<br>71.96 | 9.22<br>9.16 | 4.00<br>4.21 |
| 16 | $-n-C_4H_9$ | 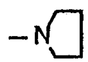 | 154.5 - 155.5 " | 2960, 2940, 2880, 2800- 2200, 1670, 1605 | 0.63 - 4.13 (24H, m), 4.33 - 4.87 (1H, m), 7.20 (2H, d), 8.07 (2H, d), 12.47 (1H, br) | $C_{20}H_{29}NO \cdot HCl$ | 71.51<br>71.69 | 9.00<br>9.13 | 4.17<br>4.23 |
| 17 | " | 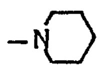 | 165.0 - 166.0 " | 2960, 2930, 2870, 2800- 2300, 1665, 1600 | 0.60 - 4.20 (26H, m), 4.30 - 4.87 (1H, m), 7.17 (2H, d), 8.00 (2H, d), 12.10 (1H, br) | $C_{21}H_{31}NO \cdot HCl$ | 72.08<br>72.00 | 9.22<br>9.40 | 4.00<br>3.98 |
| 18 | $-n-C_5H_{11}$ | 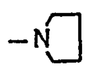 | 144.0 - 145.0 " | 2960, 2930, 2850, 2800- 2300, 1670, 1600 | 0.57 - 4.13 (26H, m), 4.30 - 4.83 (1H, m), 7.18 (2H, d), 8.05 (2H, d), 12.48 (1H, br) | $C_{21}H_{31}NO \cdot HCl$ | 72.08<br>72.14 | 9.22<br>9.37 | 4.00<br>4.25 |
| 19 | " | 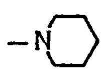 | 154.0 - 155.0 " | 2950, 2920, 2850, 2800- 2300, 1660, 1595 | 0.60 - 4.13 (28H, m), 4.30 - 4.90 (1H, m), 7.17 (2H, d), 8.03 (2H, d), 12.10 (1H, br) | $C_{22}H_{33}NO \cdot HCl$ | 72.60<br>72.59 | 9.42<br>9.38 | 3.85<br>3.72 |

Table-9 continued

| | | | | | | | | | |
|---|---|---|---|---|---|---|---|---|---|
| 20 | $-n-C_6H_{13}$ | 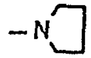 | 109.0-110.5<br>tetrahydrofuran-iospropylether | 2960, 2930, 2850, 2750-2200, 1670, 1600 | 0.57-4.10(28H, m), 4.30-4.80(1H, m), 7.13(2H, d), 8.02(2H, d), 12.47(1H, br) | $C_{22}H_{33}NO \cdot HCl$ | 72.60<br>72.72 | 9.42<br>9.45 | 3.85<br>3.74 |
| 21 | 〃 | 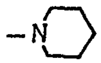 | 128.0-129.0<br>〃 | 2960, 2930, 2860, 2800-2300, 1670, 1600 | 0.53-4.13(30H, m), 4.30-4.87(1H, m), 7.15(2H, d), 7.98(2H, d), 12.13(1H, br) | $C_{23}H_{35}NO \cdot HCl$ | 73.08<br>72.94 | 9.60<br>9.54 | 3.71<br>3.66 |
| 22 | $-CH_3$ | 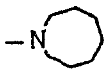 | 167.0-168.0<br>(dec.)<br>isopropanol | 2920, 2850, 2800-2200, 1665, 1600 | 0.48-2.42(15H, m), 1.30(3H, d), 2.45-4.25(6H, m), 4.28-4.95(1H, m), 7.15(2H, d), 7.98(2H, d), 12.05(1H, br) | $C_{20}H_{29}NO \cdot HCl$ | 71.51<br>71.64 | 9.00<br>8.96 | 4.17<br>4.20 |
| 23 | $-CH_2CH_3$ | 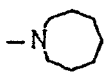 | 156.0-158.0<br>〃 | 2960, 2930, 2870, 2860, 2800-2200, 1670, 1610 | 0.47-5.07(24H, m), 0.92(3H, t), 7.15(2H, d), 8.00(2H, d), 11.97(1H, br) | $C_{21}H_{31}NO \cdot HCl$ | 72.08<br>71.96 | 9.22<br>9.31 | 4.00<br>4.13 |
| 24 | $-CH_3$ | 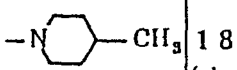 | 184.0-185.0<br>(dec.)<br>〃 | 2940, 2900, 2860, 2750-2200, 1665, 1600 | 0.67-3.93(19H, m), 1.23(3H, d), 4.27-4.83(1H, m), 7.05(2H, d), 7.87(2H, d), 12.26(1H, br) | $C_{19}H_{27}NO \cdot HCl$ | 70.90<br>70.87 | 8.77<br>8.69 | 4.35<br>4.41 |
| 25 | 〃 | 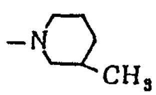 | 190.5-191.0<br>(dec.)<br>〃 | 2960, 2940, 2870, 2800-2200, 1670, 1600 | 0.57-4.14(16H, m), 0.79(3H, d), 1.30(3H, d), 4.24-4.88(1H, m), 7.10(2H, d), 7.93(2H, d), 12.11(1H, br) | $C_{19}H_{27}NO \cdot HCl$ | 70.90<br>70.79 | 8.77<br>8.74 | 4.35<br>4.38 |
| 26 | $-CH_3$ | 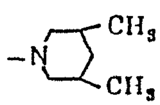 | 189.0-190.0<br>(dec.)<br>〃 | 2950, 2930, 2870, 2800-2200, 1670, 1600 | 0.56-4.12(21H, m), 1.29(3H, d), 4.29-4.92(1H, m), 7.13(2H, d), 7.96(2H, d), 12.22(1H, br) | $C_{20}H_{29}NO \cdot HCl$ | 71.51<br>71.48 | 9.00<br>8.87 | 4.17<br>4.02 |

Table-9 continued

| | | | | | | | | | |
|---|---|---|---|---|---|---|---|---|---|
| 27 | 〃 | 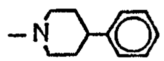 | 179.0 - 181.0 (dec.) 〃 | 2940, 2880, 2800-2200, 1690, 1610 | 0.67 - 4.20 (16H, m), 4.33 - 4.90 (1H, m), 7.13 (2H, d), 7.20 (5H, s), 7.98 (2H, d), 12.40 (1H, br) | $C_{21}H_{29}NO \cdot HCl$ | 75.08<br>75.18 | 7.88<br>7.65 | 3.65<br>3.56 |
| 28 | 〃 | 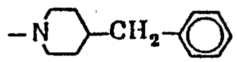 | 178.0 - 180.0 (dec.) 〃 | 2950, 2920, 2800-2200, 1665, 1605 | 0.67 - 4.07 (18H, m), 1.27 (3H, d), 4.27 - 4.87 (1H, m), 6.87 - 7.40 (7H, m), 7.95 (2H, d), 12.23 (1H, br) | $C_{25}H_{31}NO \cdot HCl$ | 75.45<br>75.39 | 8.11<br>8.04 | 3.52<br>3.46 |
| 29 | 〃 | 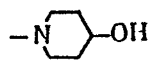 | 140.0 - 142.0 tetrahydrofuran | 3260, 2970, 2920, 2880, 2750-2200, 1670, 1600 | 0.67 - 4.70 (18H, m), 1.27 (3H, d), 7.13 (2H, d), 7.92 (2H, d), 12.37 (1H, br) | $C_{18}H_{25}NO_3 \cdot HCl$ | 66.76<br>66.85 | 8.09<br>8.01 | 4.33<br>4.42 |
| 30 | 〃 | 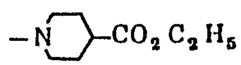 | 164.0 - 168.0 isopropanol | 2980, 2940, 2800-2200, 1720, 1670, 1600 | 0.63 - 4.30 (19H, m), 1.22 (3H, d), 1.27 (3H, t), 7.14 (2H, d), 7.97 (2H, d), 12.03 (1H, br) | $C_{21}H_{29}NO_3 \cdot HCl$ | 66.39<br>66.45 | 7.96<br>7.86 | 3.69<br>3.57 |
| 31 | $-CH_3$ | 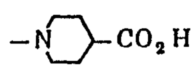 | 172.0 - 174.0 〃 | 2940, 2870, 2750-2200, 1725, 1660, 1600 | * 0.62 - 1.45 (4H, m), 1.26 (3H, d), 1.52 - 4.05 (12H, m), 4.15 - 4.72 (1H, m), 7.17 (2H, d), 7.95 (2H, d), 11.15 (2H, br) | $C_{19}H_{25}NO_3 \cdot HCl$ | 64.86<br>64.95 | 7.45<br>7.49 | 3.98<br>3.84 |

Table-9 continued

| | | | | | | | | | |
|---|---|---|---|---|---|---|---|---|---|
| 32 | $-CH(CH_3)_2$ | 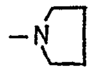 | 189.5 - 190.5 (dec.) isopropanol-tetrahydrofuran | 2970, 2900, 2880, 2800-2000, 1675, 1600 | 0.47 - 1.33 (4H, m), 0.84 (3H, d), 1.09 (3H, d), 1.63 - 4.13 (12H, m), 4.27 - 4.73 (1H, m), 7.15 (2H, d), 8.02 (2H, d), 12.30 (1H, br) | $C_{10}H_{27}NO\cdot HCl$ | 70.90<br>70.85 | 8.77<br>8.90 | 4.35<br>4.28 |
| 33 | $-CH(CH_3)_2$ | 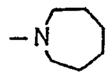 | 141.0 - 142.0 trtrahydrofuran-ethylether | 2960, 2940, 2880, 2800-2200, 1665, 1600 | 0.50 - 4.23 (20H, m), 0.81 (3H, d), 1.12 (3H, d), 4.33 - 4.77 (1H, m), 7.14 (2H, d), 7.98 (2H, d), 12.17 (1H, br) | $C_{21}H_{31}NO\cdot HCl$ | 72.08<br>71.97 | 9.22<br>9.15 | 4.00<br>4.13 |
| 34 | $-CH_2CH(CH_3)_2$ | 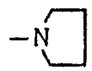 | 170.0 - 171.0 tetrahydrofuran | 2950, 2890, 2750-2000, 1665, 1600 | 0.43 - 4.20 (18H, m), 0.91 (3H, d), 1.03 (3H, d), 4.26 - 4.93 (1H, m), 7.15 (2H, d), 8.07 (2H, d), 12.45 (1H, br) | $C_{20}H_{29}NO\cdot HCl$ | 71.51<br>71.58 | 9.00<br>9.11 | 4.17<br>4.25 |
| 35 | $-CH_2CH(CH_3)_2$ | 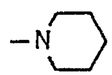 | 152.0 - 153.0 〃 | 2950, 2910, 2860, 2750-2100, 1660, 1600 | 0.57 - 4.12 (20H, m), 0.90 (3H, d), 1.10 (3H, d), 4.28 - 4.88 (1H, m), 7.12 (2H, d), 7.98 (2H, d), 12.18 (1H, br) | $C_{21}H_{31}NO\cdot HCl$ | 72.08<br>72.17 | 9.22<br>9.17 | 4.00<br>3.95 |
| 36 | 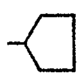 | 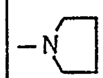 | 171.0 - 172.0 (dec.) 〃 | 2950, 2860, 2750-2000, 1670, 1600 | 0.53 - 4.13 (24H, m), 4.28 - 4.78 (1H, m), 7.17 (2H, d), 8.09 (2H, d), 12.48 (1H, br) | $C_{21}H_{29}NO\cdot HCl$ | 72.50<br>72.64 | 8.69<br>8.68 | 4.03<br>3.97 |

Table-9 continued

| | | | | | | | | | |
|---|---|---|---|---|---|---|---|---|---|
| 37 | 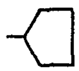 | 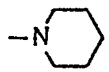 | 169.0 - 170.0 (dec.) tetrahydrofuran -ethylether | 2940, 2860, 2770-2000, 1665, 1600 | 0.50 - 4.17 (26H, m), 4.30 - 4.83 (1H, m), 7.17 (2H, d), 8.04 (2H, d), 12.17 (1H, br) | $C_{22}H_{31}NO \cdot HCl$ | 73.00<br>72.96 | 8.91<br>8.80 | 3.87<br>3.93 |
| 38 | $-CH_2-$ 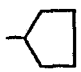 | 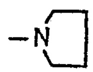 | 172.0 - 173.0 tetrahydrofuran | 2950, 2850, 2770-2200, 1660, 1600 | 0.53 - 4.17 (26H, m), 4.30 - 4.93 (1H, m), 7.19 (2H, d), 8.08 (2H, d), 12.47 (1H, br) | $C_{22}H_{31}NO \cdot HCl$ | 73.00<br>73.18 | 8.91<br>8.86 | 3.87<br>3.85 |
| 39 | $-CH_2-$ 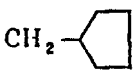 | 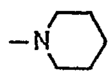 | 165.0 - 166.0 (dec.) 〃 | 2930, 2850, 2780-2100, 1650, 1595 | 0.60 - 4.13 (28H, m), 4.27 - 4.87 (1H, m), 7.17 (2H, d), 8.03 (2H, d), 12.18 (1H, br) | $C_{23}H_{33}NO \cdot HCl$ | 73.48<br>73.53 | 9.12<br>9.08 | 3.73<br>3.87 |
| 40 | 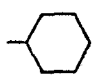 | 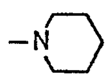 | 180.0 - 181.0 (dec.) " | 2940, 2860, 2780-2000, 1675, 1600 | 0.50 - 4.10 (28H, m), 4.23 - 4.73 (1H, m), 7.13 (2H, d), 7.96 (2H, d), 12.08 (1H, br) | $C_{23}H_{33}NO \cdot HCl$ | 73.48<br>73.40 | 9.12<br>9.06 | 3.73<br>3.83 |

* NMR($CDCl_3$ + DMSO-$d_6$)

0163537

Example 41

1-(4-Cyclopentylphenyl)-2-methyl-3-piperidino-1-propanone hydrochloride

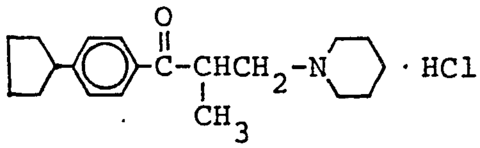

3.41 g of 4'-cyclopentylpropiophenone, 0.66 g of paraformaldehyde, 2.46 g of piperidine hydrochloride and one drop of concentrated hydrochloric acid were added to 1.5 ml of ethanol and the resulting reaction mixture was heated under reflux for 3 hours. After the reaction mixture was allowed to cool, 50 ml of chloroform was added thereto and the resulting mixture was washed three times with 30-ml portions of a saturated aqueous solution of sodium chloride. After the chloroform layer was isolated and dried over anhydrous sodium sulfate, the solvent was distilled off under reduced pressure to obtain a residue, which was recrystallized from isopropanol to obtain 4.70 g (83 %) of 1-(4-cyclopentylphenyl)-2-methyl-3-piperidino-1-propanone hydrochloride in the form of white crystals, M.P. 174.0 - 175.0°C (dec.).

IR(KBr)$cm^{-1}$: 2940, 2860, 2750 - 2200, 1670, 1600

NMR($CDCl_3$) δ ppm: 1.15 - 4.18(21H,m), 1.30(3H,d), 4.29 - 4.92(1H,m), 7.29(2H,d), 7.94(2H,d), 12.09(1H,br)

Elemental analysis for $C_{20}H_{29}NO \cdot HCl$

| | | C | H | N |
|---|---|---|---|---|
| Calcd. | % | 71.51 | 9.00 | 4.17 |
| Found | % | 71.68 | 8.95 | 4.20 |

By the application of the similar procedure as described in Example 41, the compounds of Examples 42 - 46 below were obtained.

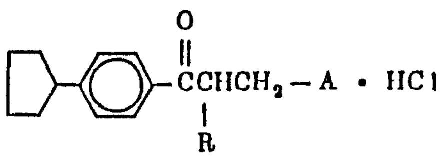

Table - 10

| Example No. | R | A | Melting point °C Recrystn. solvent | IR(KBr) $cm^{-1}$ | NMR($CDCl_3$) δ ppm | Molecular formula | Elemental analysis upper row Calcd. lower row Found | | |
|---|---|---|---|---|---|---|---|---|---|
| | | | | | | | C % | H % | N % |
| 42 | $-CH_3$ | 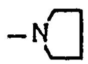 | 166.0-167.0 (dec.) isopropanol-ethylether | 2950, 2860, 2750-2200, 1675, 1600 | 1.07-4.13 (19H, m), 1.31 (3H, d), 4.23-4.90 (1H, m), 7.33 (2H, d), 7.99 (2H, d), 12.27 (1H, br) | $C_{10}H_{27}NO \cdot HCl$ | 70.90<br>70.52 | 8.77<br>8.69 | 4.35<br>4.31 |
| 43 | 〃 | 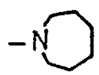 | 185.0-189.0 (dec.) isopropanol | 2950, 2930, 2860, 2750-2200, 1665, 1600 | 1.14-4.24 (23H, m), 1.29 (3H, d), 4.30-4.93 (1H, m), 7.34 (2H, d), 7.99 (2H, d), 12.14 (1H, br) | $C_{21}H_{31}NO \cdot HCl$ | 72.08<br>72.18 | 9.22<br>9.13 | 4.00<br>3.92 |
| 44 | $-C_2H_5$ | 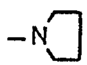 | 168.0-169.5 isopropanol-ethylether | 2970, 2940, 2880, 2800-2200, 1690, 1605 | 0.90 (3H, t), 1.33-4.13 (21H, m), 4.30-4.83 (1H, m), 7.35 (2H, d), 8.06 (2H, d), 12.33 (1H, br) | $C_{20}H_{29}NO \cdot HCl$ | 71.51<br>71.47 | 9.00<br>9.07 | 4.17<br>4.28 |
| 45 | 〃 | 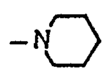 | 183.0-184.5 (dec.) " | 2970, 2880, 2800-2300, 1675, 1605 | 0.93 (3H, t), 1.30-4.17 (23H, m), 4.30-4.80 (1H, m), 7.37 (2H, d), 8.03 (2H, d), 12.07 (1H, br) | $C_{21}H_{31}NO \cdot HCl$ | 72.08<br>72.19 | 9.22<br>9.30 | 4.00<br>4.02 |
| 46 | 〃 | 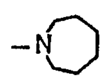 | 178.0-180.0 (dec.) isopropanol | 2960, 2870, 2800-2300, 1670, 1600 | 0.93 (3H, t), 1.27-4.28 (25H, m), 4.37-4.87 (1H, m), 7.38 (2H, d), 8.07 (2H, d), 12.13 (1H, br) | $C_{22}H_{33}NO \cdot HCl$ | 72.60<br>72.55 | 9.42<br>9.52 | 3.85<br>3.88 |

0163537

Example 47

1-(4-Cyclohexylphenyl)-2-methyl-3-(1-pyrrolidinyl)-1-propanone hydrochloride

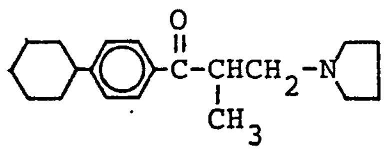 · HCl

3.65 g of 4'-cyclohexylpropiophenone, 0.66 g of paraformaldehyde, 2.17 g of pyrrolidine hydrochloride and one drop of concentrated hydrochloric acid were added to 2 ml of a solvent mixture of ethanol and isopropanol (1:1) and the resulting reaction mixture was heated under reflux for 3.5 hours. After the reaction mixture was allowed to cool, 50 ml of chloroform was added thereto and the resulting mixture was washed three times with 30-ml portions of a saturated aqueous solution of sodium chloride. After the chloroform layer was isolated and dried over anhydrous sodium sulfate, the solvent was distilled off under reduced pressure to obtain a residue, which was recrystallized from a solvent mixture of isopropanol and ethyl ether to obtain 4.37 g (77 %) of 1-(4-cyclohexylphenyl)-2-methyl-3-(1-pyrrolidinyl)-1-propanone hydrochloride in the form of white crystals, M.P. 176.0 - 177.0°C (dec.).

IR(KBr)$cm^{-1}$: 2960, 2920, 2850, 2750 - 2200, 1680, 1600

NMR($CDCl_3$) δ ppm: 1.05 - 4.08(21H,m), 1.25(3H,d), 4.18 - 4.85(1H,m), 7.25(2H,d), 7.95(2H,d), 12.28(1H,br)

Elemental analysis for $C_{20}H_{29}NO \cdot HCl$

| | | C | H | N |
|---|---|---|---|---|
| Calcd. | % | 71.51 | 9.00 | 4.17 |
| Found | % | 71.48 | 9.20 | 4.30 |

By the application of the similar procedure as described in Example 47, the compounds of Examples 48 - 52 below were obtained.

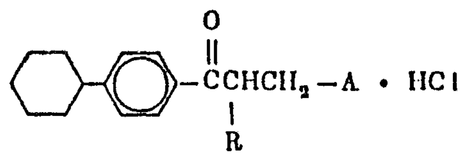

Table - 11

| Example No. | R | A | Melting point °C Recrystn. solvent | IR(KBr) cm⁻¹ | NMR($CDCl_3$) δ ppm | Molecular formula | Elemental analysis upper row Calcd. lower row Found C % | H % | N % |
|---|---|---|---|---|---|---|---|---|---|
| 48 | $-CH_3$ | 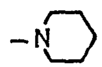 | 187.0-189.0 (dec.) isopropanol | 2920, 2850, 2750-2200, 1670, 1600 | 1.12-4.18(23H, m), 1.30(3H, d), 4.28-4.95(1H, m), 7.31(2H, d), 8.00(2H, d), 12.10(1H, br) | $C_{21}H_{31}NO\cdot HCl$ | 72.08<br>72.11 | 9.22<br>9.31 | 4.00<br>4.24 |
| 49 | " | 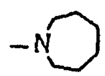 | 177.0-178.0 (dec.) " | 2920, 2840, 2750-2200, 1665, 1600 | 1.17-4.23(25H, m), 1.31(3H, d), 4.30-4.90(1H, m), 7.30(2H, d), 8.00(2H, d), 12.17(1H, br) | $C_{22}H_{33}NO\cdot HCl$ | 72.60<br>72.59 | 9.42<br>9.54 | 3.85<br>3.84 |
| 50 | $-C_2H_5$ | 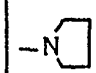 | 169.0-179.0 (dec.) " | 2960, 2920, 2850, 2750-2200, 1680, 1595 | 0.67-4.10(23H, m), 0.87(3H, t), 4.20-4.73(1H, m), 7.27(2H, d), 7.97(2H, d), 12.13(1H, br) | $C_{21}H_{31}NO\cdot HCl$ | 72.08<br>71.96 | 9.22<br>9.09 | 4.00<br>4.27 |
| 51 | " | 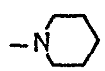 | 179.0-185.0 (dec.) " | 2930, 2850, 2800-2200, 1670, 1600 | 0.63-4.13(25H, m), 0.93(3H, t), 4.27-4.80(1H, m), 7.30(2H, d), 7.99(2H, d), 12.17(1H, br) | $C_{22}H_{33}NO\cdot HCl$ | 72.60<br>72.56 | 9.42<br>9.36 | 3.85<br>3.75 |
| 52 | " | 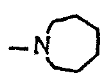 | 180.0-186.0 (dec.) " | 2960, 2920, 2860, 2800-2300, 1670. 1600 | 0.63-4.23(27H, m), 0.93(3H, t), 4.30-4.80(1H, m), 7.30(2H, d), 8.00(2H, d), 12.17(1H, br) | $C_{23}H_{35}NO\cdot HCl$ | 73.08<br>73.12 | 9.60<br>9.59 | 3.71<br>3.64 |

0163537

Example 53

2-Ethyl-1-(3,4-methylenedioxyphenyl)-3-piperidino-1-propanone hydrochloride

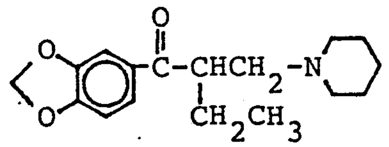 ·HCl

3.00 g of the 3',4'-methylenedioxybutyrophenone prepared in Reference Example 4, 0.63 g of paraformaldehyde, 2.29 g of piperidine hydrochloride and one drop of concentrated hydrochloric acid were added to 2.0 ml of isopropanol and the resulting reaction mixture was heated under reflux for 3 hours. After the reaction mixture was allowed to cool, 50 ml of chloroform was added thereto and the resulting mixture was washed three times with 30-ml portions of a saturated aqueous solution of sodium chloride. After the chloroform layer was dried over anhydrous sodium sulfate, the solvent was distilled off under reduced pressure to obtain a residue, which was recrystallized from tetrahydrofuran to obtain 4.12 g (81 %) of 2-ethyl-1-(3,4-methylenedioxyphenyl)-3-piperidino-1-propanone hydrochloride in the form of white crystals, M.P. 175.0 - 177.0°C.

IR(KBr)$cm^{-1}$: 2940, 2860, 2760 - 2150, 1660, 1600, 1490, 1440, 1260

NMR($CDCl_3$) δ ppm: 0.92(3H,t), 1.18 - 4.13(14H,m), 4.20 - 4.73(1H,m), 6.03(2H,s), 6.85(1H,d), 7.45(1H,d), 7.73(1H,dd), 12.05(1H,br)

Elemental analysis for $C_{17}H_{23}NO_3$•HCl

| | | C | H | N |
|---|---|---|---|---|
| Calcd. | % | 62.67 | 7.43 | 4.30 |
| Found | % | 62.59 | 7.51 | 4.38 |

Example 54

2-Ethyl-1-(3,4-methylenedioxyphenyl)-3-piperidino-1-propanone

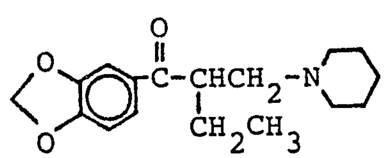

1.50 g of the 2-ethyl-1-(3,4-methylenedioxyphenyl)-3-piperidino-1-propanone hydrochloride obtained in Example 53

was dissolved in 25 ml of water and the resulting solution was alkalified with a 20 % aqueous solution of sodium hydroxide. The oily material so separated was extracted with ethyl ether and washed three times with a saturated aqueous solution of sodium chloride. The ethyl ether layer was isolated, dried over anhydrous sodium sulfate and then distilled under reduced pressure to remove the solvent. The resulting oily residue was purified by silica gel column chromatography [using an eluent composed of chloroform and ethanol (20:1)] to obtain 1.22 g (92 %) of 2-ethyl-1-(3,4-methylenedioxyphenyl)-3-piperidino-1-propanone in the form of an oily material.

IR(Neat)$cm^{-1}$: 2960, 2930, 2850, 2800, 1670, 1605, 1500, 1490, 1440, 1260

NMR($CDCl_3$) δ ppm: 0.70 - 3.03(17H,m), 3.23 - 3.80(1H,m), 6.02(2H,s), 6.82(1H,d), 7.43(1H,d), 7.56(1H,dd)

Example 55

2-Ethyl-1-(3,4-methylenedioxyphenyl)-3-piperidino-1-propanone nitrate

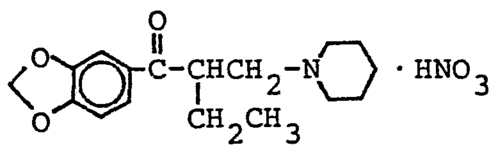

1.00 g of the 2-ethyl-1-(3,4-methylenedioxyphenyl)-3-piperidino-1-propanone obtained in Example 54 was dissolved in 10 ml of methanol and the resulting solution was acidified with 30 % nitric acid. Then, the solvent was distilled off under reduced pressure to obtain a residue, to which ethyl ether was added. The resulting mixture was stirred vigorously. The crystals so precipitated were collected by filtration and recrystallized from a solvent mixture of isopropanol and tetrahydrofuran to obtain 0.95 g (78 %) of 2-ethyl-1-(3,4-methylenedioxyphenyl)-3-piperidino-1-propanone nitrate, M.P. 167.0 - 169.5°C (dec.).

IR(KBr)$cm^{-1}$: 3050, 2960, 2880, 2830 - 2200, 1660, 1600, 1440, 1380, 1345, 1250

NMR($CDCl_3$) δ ppm: 0.90(3H,t), 1.10 - 4.33(15H,m), 6.03(2H,s), 6.85(1H,d), 7.40(1H,d), 7.63(1H,dd), 10.90(1H,br)

Elemental analysis for $C_{17}H_{23}NO_3 \cdot HNO_3$

BAD ORIGINAL

| | | C | H | N |
|---|---|---|---|---|
| Calcd. | % | 57.94 | 6.87 | 7.95 |
| Found | % | 57.88 | 6.90 | 8.03 |

Example 56

2-Ethyl-1-(3,4-methylenedioxyphenyl)-3-(1-pyrrolidinyl)-1-propanone hydrochloride

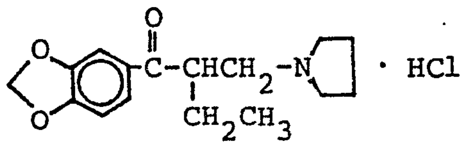

3.00 g of the 3',4'-methylenedioxybutyrophenone prepared in Reference Example 4, 0.63 g of paraformaldehyde, 2.03 g of pyrrolidine hydrochloride and one drop of concentrated hydrochloric acid were added to 2 ml of ethanol and the resulting reaction mixture was heated under reflux for 4 hours. After the reaction mixture was allowed to cool, 50 ml of chloroform was added thereto and the resulting mixture was washed three times with 30-ml portions of a saturated aqueous solution of sodium chloride. After the chloroform layer was dried over anhydrous sodium sulfate, the solvent was distilled off under reduced pressure to obtain a residue, which was recrystallized from tetrahydrofuran to obtain 3.89 g (80 %) of 2-ethyl-1-(3,4-methylenedioxyphenyl)-3-(1-pyrrolidinyl)-1-propanone hydrochloride in the form of white crystals, M.P. 157.0 - 159.0°C.

IR(KBr)$cm^{-1}$: 2960, 2880, 2800 - 2150, 1670, 1600, 1495, 1440, 1270

NMR($CDCl_3$) δ ppm: 0.90(3H,t), 1.37 - 4.20(12H,m), 4.23 - 4.77(1H,m), 6.06(2H,s), 6.89(1H,d), 7.50(1H,d), 7.82(1H,dd), 12.45(1H,br)

Elemental analysis for $C_{16}H_{21}NO_3 \cdot HCl$

| | | C | H | N |
|---|---|---|---|---|
| Calcd. | % | 61.63 | 7.11 | 4.49 |
| Found | % | 61.56 | 7.08 | 4.59 |

Example 57

2-Methyl-1-(3,4-methylenedioxyphenyl)-3-piperidino-1-propanone hydrochloride

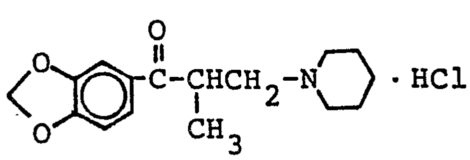

3.01 g of the 3',4'-methylenedioxypropiophenone prepared in Reference Example 18, 0.66 g of paraformaldehyde, 2.46 g of piperidine hydrochloride and one drop of concentrated hydrochloric acid were added to 2 ml of a solvent mixture of isopropanol and ethanol (1:1) and the resulting reaction mixture was heated under reflux for 4 hours. After the reaction mixture was allowed to cool, 40 ml of chloroform was added thereto and the resulting mixture was washed three times with 30-ml portions of a saturated aqueous solution of sodium chloride. After the chloroform layer was dried over anhydrous sodium sulfate, the solvent was distilled off under reduced pressure to obtain a residue, which was recrystallized from isopropanol to obtain 3.84 g (73 %) of 2-methyl-1-(3,4-methylenedioxyphenyl)-3-piperidino-1-propanone hydrochloride in the form of white crystals, M.P. 189.0 - 190.0°C (dec.).

IR(KBr)$cm^{-1}$: 2970, 2940, 2770 - 2150, 1665, 1595, 1480, 1430, 1250

NMR($CDCl_3$) δ ppm: 0.95 - 4.15(12H,m), 1.29(3H,d), 4.18 - 4.88(1H,m), 6.05(2H,s), 6.85(1H,d), 7.44(1H,d), 7.73(1H,dd), 11.98(1H,br)

Elemental analysis for $C_{16}H_{21}NO_3 \cdot HCl$

| | | C | H | N |
|---|---|---|---|---|
| Calcd. | % | 61.63 | 7.11 | 4.19 |
| Found | % | 61.49 | 7.19 | 4.48 |

Example 58

(+)-2-Ethyl-1-(3,4-methylenedioxyphenyl)-3-piperidino-1-propanone hydrochloride

(+)-isomer 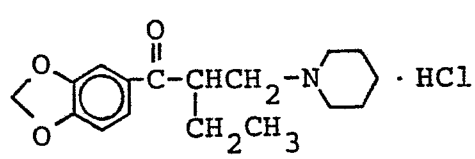

(a) (+)-2-Ethyl-1-(3,4-methylenedioxyphenyl)-3-piperidino-1-propanone (-)-N-acetyl-D-α-phenylglycinate

37.0 g of 2-ethyl-1-(3,4-methylenedioxyphenyl)-3-piperidino-1-propanone hydrochloride prepared according to the procedure of Example 53 was dissolved in 400 ml of water and the resulting solution was alkalified with a 10 % aqueous solution of sodium hydroxide, followed by vigorous stirring. The oily

BAD ORIGINAL

material so separated was extracted three times with 100-ml portions of benzene. The benzene layer was washed with water, dried over anhydrous sodium sulfate and then distilled under reduced pressure to remove the solvent. The resulting oily residue was dissolved in 100 ml of acetone, and 21.9 g of (-)-N-acetyl-D-α-phenylglycine was added thereto and dissolved therein by the application of heat. After the solution was allowed to cool, the solvent was distilled off under reduced pressure to obtain an oily residue. A solvent mixture of isopropyl ether (150 ml) and acetone (100 ml) was added thereto and dissolved therein by the application of heat, and the resulting solution was allowed to stand in the cold. The crystals so precipitated were collected by filtration and recrystallized twice from ethyl acetate to obtain 20.6 g of (+)-2-ethyl-1-(3,4-methylenedioxyphenyl)-3-piperidino-1-propanone (-)-N-acetyl-D-α-phenylglycinate, $[\alpha]_D^{25}$ -63.2° (C=1, methanol), M.P. 128.0 - 128.5°C.

IR(KBr)$cm^{-1}$: 3240, 3210, 3060, 2950, 2920, 2730 - 2000, 1665, 1600, 1545

NMR($CDCl_3$) δ ppm: 0.72(3H,t), 1.17 - 1.90(8H,m), 2.03(3H,s), 2.43 - 4.27(7H,m), 5.28(1H,d), 6.03(2H,s), 6.78(1H,d), 6.97 - 7.67(8H,m), 13.55(1H,br)

Elemental analysis for $C_{27}H_{34}N_2O_6$

| | | C | H | N |
|---|---|---|---|---|
| Calcd. | % | 67.20 | 7.10 | 5.81 |
| Found | % | 67.16 | 7.21 | 5.81 |

(b) (+)-2-Ethyl-1-(3,4-methylenedioxyphenyl)-3-piperidino-1-propanone hydrochloride

19 g of the (+)-2-ethyl-1-(3,4-methylenedioxyphenyl)-3-piperidino-1-propanone (-)-N-acetyl-D-α-phenylglycinate obtained in (a) was dissolved in 300 ml of water and the resulting solution was alkalified with a 10 % aqueous solution of sodium hydroxide. The oily material so separated was extracted three times with 100-ml portions of benzene. The benzene layer was washed with water, dried over anhydrous sodium sulfate and then

BAD ORIGINAL

concentrated under reduced pressure to a volume of about 50 ml. This solution was acidified by the addition of benzene saturated with hydrogen chloride and then distilled under reduced pressure to remove the solvent. The resulting solid residue was recrystallized from tetrahydrofuran to obtain 9.1 g of (+)-2-ethyl-1-(3,4-methylenedioxyphenyl)-3-piperidino-1-propanone hydrochloride, $[\alpha]_D^{24}$ +5.9°(C=2, methanol), M.P. 169.5 - 170.0°C.

IR(KBr)$cm^{-1}$: 3120, 3090, 2950, 2890, 2800 - 2000, 1670, 1600

NMR($CDCl_3$) δ ppm: 0.95(3H,t), 1.17 - 4.10(14H,m), 4.27 - 4.77(1H,m), 6.10(2H,s), 6.90(1H,d), 7.50(1H,d), 7.79(1H,dd), 12.08(1H,br)

Elemental analysis for $C_{17}H_{23}NO_3 \cdot HCl$

| | | C | H | N |
|---|---|---|---|---|
| Calcd. | % | 62.67 | 7.43 | 4.30 |
| Found | % | 62.69 | 7.50 | 4.23 |

Example 59

(-)-2-Ethyl-1-(3,4-methylenedioxyphenyl)-3-piperidino-1-propanone hydrochloride

(-)-isomer 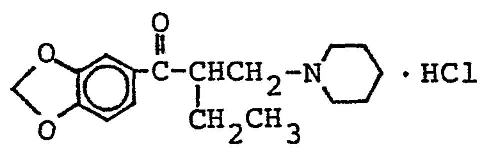

(a) (-)-2-Ethyl-1-(3,4-methylenedioxyphenyl)-3-piperidino-1-propanone (+)-N-acetyl-L-α-phenylglycinate

The crystallization and recrystallization mother liquors obtained in Example 58(a) were combined and concentrated under reduced pressure. The resulting residue was dissolved in 300 ml of water and this solution was alkalified with a 10 % aqueous solution of sodium hydroxide. The oily material so separated was extracted three times with 100-ml portions of benzene. The benzene layer was washed with water, dried over anhydrous sodium sulfate and then distilled under reduced pressure to remove the solvent. Thus, there was obtained 18.1 g of an oily material containing a large amount of the (-)-isomer. This oily material was dissolved in 200 ml of acetone, and 12.1 g of (+)-N-acetyl-L-α-phenylglycine was added thereto and dissolved therein by the application of heat. After the solution was

allowed to cool, the solvent was distilled off under reduced pressure and the resulting solid residue was recrystallized twice from ethyl acetate to obtain 12.3 g of (-)-2-ethyl-1-(3,4-methylenedioxyphenyl)-3-piperidino-1-propanone (+)-N-acetyl-L-α-phenylglycinate, $[\alpha]_D^{25}$ +62.8°(C=1.0, methanol), M.P. 127.0 - 128.0°C.

IR(KBr)$cm^{-1}$: 3240, 3210, 3060, 2950, 2930, 2730 - 2000, 1665, 1600, 1550

NMR($CDCl_3$) δ ppm: 0.72(3H,t), 1.17 - 1.93(8H,m), 2.02(3H,s), 2.43 - 4.23(7H,m), 5.28(1H,d), 6.05(2H,s), 6.79(1H,d), 6.97 - 7.67(8H,m), 13.53(1H,br)

Elemental analysis for $C_{27}H_{34}N_2O_6$

| | | C | H | N |
|---|---|---|---|---|
| Calcd. | % | 67.20 | 7.10 | 5.81 |
| Found | % | 67.26 | 7.10 | 5.73 |

(b) (-)-2-Ethyl-1-(3,4-methylenedioxyphenyl)-3-piperidino-1-propanone hydrochloride

5.0 g of the (-)-2-ethyl-1-(3,4-methylenedioxyphenyl)-3-piperidino-1-propanone (+)-N-acetyl-L-α-phenylglycinate obtained in (a) was dissolved in 50 ml of water and the resulting solution was alkalified with a 10 % aqueous solution of sodium hydroxide. The oily material so separated was extracted three times with 50-ml portions of benzene. The combined benzene layer was washed with water, dried over anhydrous sodium sulfate and then concentrated under reduced pressure to a volume of about 50 ml. This solution was acidified by the addition of benzene saturated with hydrgoen chloride and then distilled under reduced pressure to remove the solvent. The resulting solid residue was recrystallized from tetrahydrofuran to obtain 2.5 g of (-)-2-ethyl-1-(3,4-methylenedioxyphenyl)-3-piperidino-1-propanone hydrochloride, $[\alpha]_D^{24}$ -5.5°(C=2.0, methanol), M.P. 169.5 - 170.0°C.

IR(KBr)$cm^{-1}$: 3120, 3090, 2950, 2880, 2800 - 2000, 1670, 1600

NMR($CDCl_3$) δ ppm: 0.93(3H,t), 1.17 - 4.07(14H,m), 4.27 - 4.73(1H,m), 6.08(2H,s), 6.90(1H,d), 7.50(1H,d), 7.78(1H,dd), 12.17(1H,br)

Elemental analysis for $C_{17}H_{23}NO_3 \cdot HCl$

| | | C | H | N |
|---|---|---|---|---|
| Calcd. | % | 62.67 | 7.43 | 4.30 |
| Found | % | 62.71 | 7.40 | 4.32 |

By the application of the similar procedure as described in Example 53, the compounds of Examples 60 - 84 below were obtained.

Table - 12 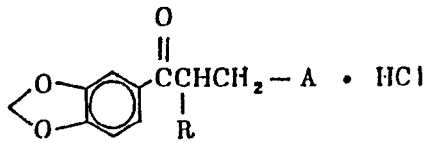

| Example No. | $R_1$ | A | Melting point °C Recrystn. solvent | IR(KBr) $cm^{-1}$ | NMR($CDCl_3$) δ ppm | Molecular formula | Elemental analysis upper row Calcd. lower row Found | | |
|---|---|---|---|---|---|---|---|---|---|
| | | | | | | | C % | H % | N % |
| 60 | $-CH_3$ | 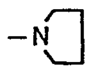 | 170.0 - 171.0 (dec.) isopropanol-ethylether | 2980, 2900, 2800, 2780-2200, 1670, 1610, 1480, 1430, 1260 | 1.29 (3H, d), 1.51 - 4.08 (10H, m), 4.15 - 4.75 (1H, m), 6.07 (2H, s), 6.86 (1H, d), 7.45 (1H, d), 7.73 (1H, dd), 12.12 (1H, br) | $C_{15}H_{19}NO_3 \cdot HCl$ | 60.50<br>60.41 | 6.77<br>6.85 | 4.70<br>4.59 |
| 61 | 〃 | 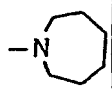 | 171.0 - 172.0 (dec.) isopropanol | 2950, 2870, 2800-2250, 1680, 1610, 1485, 1430, 1255 | 1.05 - 4.23 (14H, m), 1.26 (3H, d), 4.25 - 4.88 (1H, m), 6.05 (2H, s), 6.87 (1H, d), 7.46 (1H, d), 7.75 (1H, dd), 12.08 (1H, br) | $C_{17}H_{23}NO_3 \cdot HCl$ | 62.67<br>62.53 | 7.43<br>7.37 | 4.30<br>4.41 |
| 62 | 〃 | 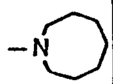 | 153.5 - 154.5 (dec.) isopropanol-tetrahydrofuran | 2930, 2860, 2800-2200, 1675, 1610, 1480, 1430, 1250 | 0.93 - 4.20 (16H, m), 1.28 (3H, d), 4.23 - 4.87 (1H, m), 6.05 (2H, s), 6.85 (1H, d), 7.44 (1H, d), 7.73 (1H, dd), 11.95 (1H, br) | $C_{18}H_{25}NO_3 \cdot HCl$ | 63.61<br>63.55 | 7.71<br>7.80 | 4.12<br>4.24 |

Table-12 continued

| | | | | | | | | | |
|---|---|---|---|---|---|---|---|---|---|
| 63 | $-C_2H_5$ | 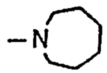 | 143.0 - 145.0 tetrahydrofuran | 2960, 2930, 2860, 2800-2150, 1665, 1600, 1490, 1440, 1250 | 0.95 (3H, t), 1.32-4.85 (17H, m), 6.10 (2H, s), 6.90 (1H, d), 7.50 (1H, d), 7.80 (1H, dd), 12.07 (1H, br) | $C_{18}H_{25}NO_3 \cdot HCl$ | 63.61<br>63.64 | 7.71<br>7.69 | 4.12<br>4.24 |
| 64 | // | 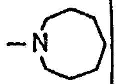 | 87.0 - 89.0<br>" | 2960, 2920, 2850, 2800-2150, 1660, 1490, 1440, 1250 | 0.90 (3H, t), 1.23-4.20 (18H, m), 4.23-4.77 (1H, m), 6.05 (2H, s), 6.87 (1H, d), 7.47 (1H, d), 7.78 (1H, dd), 12.03 (1H, br) | $C_{19}H_{27}NO_3 \cdot HCl$ | 64.49<br>64.58 | 7.98<br>7.84 | 3.96<br>3.92 |
| 65 | $-n-C_3H_7$ | 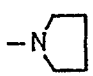 | 148.0 - 149.0<br>" | 2950, 2920, 2860, 2750-2150, 1665, 1605, 1490, 1430, 1260 | 0.62 - 4.21 (17H, m), 4.25 - 4.82 (1H, m), 6.15 (2H, s), 6.95 (1H, d), 7.57 (1H, d), 7.88 (1H, dd), 12.23 (1H, br) | $C_{17}H_{23}NO_3 \cdot HCl$ | 62.67<br>62.57 | 7.43<br>7.39 | 4.30<br>4.27 |
| 66 | // | 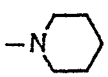 | 150.0 - 152.0<br>" | 2960, 2930, 2860, 2750-2150, 1660, 1600, 1490, 1440, 1260 | 0.60 - 4.22 (19H, m), 4.23 - 4.87 (1H, m), 6.13 (2H, s), 6.90 (1H, d), 7.50 (1H, d), 7.81 (1H, dd), 11.90 (1H, br) | $C_{18}H_{25}NO_3 \cdot HCl$ | 63.61<br>63.71 | 7.71<br>7.78 | 4.12<br>4.32 |

Table-12 continued

| | | | | | | | | | |
|---|---|---|---|---|---|---|---|---|---|
| 67 | $-iso-C_3H_7$ | 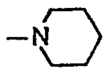 | 200.0-202.0 (dec.) isopropanol-tetrahydrofuran | 2960,2900, 2800-2100, 1665,1605, 1490,1440, 1250 | 0.82(3H,d), 1.16(3H,d), 1.37-4.15(13H,m), 4.25-4.68(1H,m), 6.08(2H,s), 6.88(1H,d), 7.48(1H,d), 7.67(1H,dd), 11.95(1H,br) | $C_{18}H_{25}NO_3 \cdot HCl$ | 63.61<br>63.49 | 7.71<br>7.83 | 4.12<br>4.20 |
| 68 | $-n-C_4H_9$ | 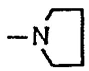 | 129.0-130.0 chloroform-ethylether | 2960,2930, 2870,2800-2200,1670, 1610,1490, 1440,1250 | 0.48-4.12(19H,m), 4.25-4.77(1H,m), 6.07(2H,s), 6.88(1H,d), 7.47(1H,d), 7.80(1H,dd), 12.40(1H,br) | $C_{18}H_{25}NO_3 \cdot HCl$ | 63.61<br>63.57 | 7.71<br>7.65 | 4.12<br>4.27 |
| 69 | 〃 | 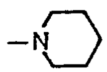 | 141.0-142.0 trtrahydrofuran | 2960,2950, 2860,2800, 2770-2150, 1670,1605, 1490,1435, 1240 | 0.53-4.13(21H,m), 4.23-4.77(1H,m), 6.05(2H,s), 6.88(1H,d), 7.47(1H,d), 7.77(1H,dd), 12.13(1H,br) | $C_{19}H_{27}NO_3 \cdot HCl$ | 64.49<br>64.54 | 7.98<br>7.86 | 3.96<br>4.06 |
| 70 | $-iso-C_4H_9$ | 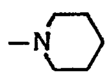 | 178.0-179.0 (dec.) 〃 | 2950,2900, 2800-2100, 1660,1600, 1485,1440, 1260 | 0.77-4.07(15H,m), 0.92(3H,d), 1.10(3H,d), 4.30-4.80(1H,m), 6.03(2H,s), 6.87(1H,d), 7.47(1H,d), 7.80(1H,dd), 12.30(1H,br) | $C_{19}H_{27}NO_3 \cdot HCl$ | 64.49<br>64.53 | 7.98<br>7.88 | 3.96<br>3.86 |

Table-12 continued

| | | | | | | | | | |
|---|---|---|---|---|---|---|---|---|---|
| 71 | $-n-C_5H_{11}$ | 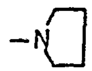 | 132.0 - 134.0 chloroform-ethylether | 2950, 2920, 2850, 2800-2200, 1665, 1600, 1490, 1440, 1255 | 0.50 - 4.13 (21H, m), 4.20 - 4.80 (1H, m), 6.07 (2H, s), 6.88 (1H, d), 7.50 (1H, d), 7.83 (1H, dd), 12.43 (1H, br) | $C_{19}H_{27}NO_3 \cdot HCl$ | 64.49<br>64.37 | 7.98<br>7.89 | 3.96<br>3.79 |
| 72 | // | 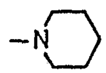 | 154.0 - 155.0 tetrahydrofuran | 2950, 2930, 2860, 2750-2100, 1670, 1600, 1490, 1440, 1250 | 0.56 - 4.16 (23H, m), 4.27 - 4.79 (1H, m), 6.09 (2H, s), 6.91 (1H, d), 7.49 (1H, d), 7.80 (1H, dd), 12.11 (1H, br) | $C_{20}H_{29}NO_3 \cdot HCl$ | 65.29<br>65.38 | 8.22<br>8.16 | 3.81<br>3.94 |
| 73 | $-n-C_6H_{13}$ | 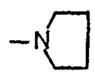 | 70.0 - 72.0 tetrahydrofuran-ethylether | 2970, 2940, 2860, 2780-2200, 1670, 1605, 1490, 1440, 1250 | 0.55 - 4.12 (23H, m), 4.22 - 4.78 (1H, m), 6.07 (2H, s), 6.91 (1H, d), 7.52 (1H, d), 7.87 (1H, dd), 12.48 (1H, br) | $C_{20}H_{29}NO_3 \cdot HCl$ | 65.29<br>65.16 | 8.22<br>8.35 | 3.81<br>3.90 |
| 74 | // | 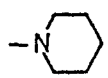 | 118.0 - 120.0 " | 2950, 2930, 2860, 2780-2200, 1670, 1600, 1490, 1440, 1250 | 0.58 - 4.15 (25H, m), 4.28 - 4.78 (1H, m), 6.07 (2H, s), 6.88 (1H, d), 7.48 (1H, d), 7.78 (1H, dd), 12.18 (1H, br) | $C_{21}H_{31}NO_3 \cdot HCl$ | 66.04<br>66.16 | 8.45<br>8.50 | 3.67<br>3.74 |

Table-12 continued

| | | | | | | | | | |
|---|---|---|---|---|---|---|---|---|---|
| 75 | 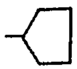 | 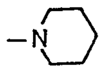 | 180.0-182.0 (dec.) tetrahydrofuran | 2940, 2850, 2800-2100, 1655, 1600, 1490, 1440, 1250 | 0.90-4.07(21H, m), 4.23-4.73(1H, m), 6.02(2H, s), 6.83(1H, d), 7.45(1H, d), 7.78(1H, dd), 12.23(1H, br) | $C_{20}H_{27}NO_3 \cdot HCl$ | 65.65<br>65.58 | 7.71<br>7.85 | 3.83<br>3.81 |
| 76 | $-CH_2-$ 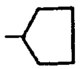 | 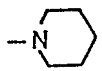 | 175.0-176.0<br>" | 2940, 2850, 2800-2100, 1660, 1600, 1490, 1440, 1260 | 0.50-4.10(23H, m), 4.23-4.87(1H, m), 6.07(2H, s), 6.87(1H, d), 7.47(1H, d), 7.82(1H, dd), 12.17(1H, br) | $C_{21}H_{29}NO_3 \cdot HCl$ | 66.39<br>66.45 | 7.96<br>7.82 | 3.69<br>3.79 |
| 77 | 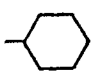 | 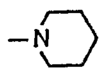 | 192.0-195.0 (dec.) isopropanol-ethylether | 2940, 2860, 2780-2200, 1670, 1600, 1440, 1250 | 0.67-4.13(23H, m), 4.27-4.63(1H, m), 6.07(2H, s), 6.87(1H, d), 7.47(1H, d), 7.75(1H, dd), 12.12(1H, br) | $C_{21}H_{29}NO_3 \cdot HCl$ | 66.39<br>66.37 | 7.96<br>7.80 | 3.69<br>3.57 |
| 78 | $-C_2H_5$ | 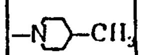 | 93.0-95.0 tetrahydrofuran -isopropylether | 2970, 2940, 2880, 2820-2200, 1670, 1600, 1490, 1440, 1250 | 0.63-1.27(6H, m), 1.27-4.10(13H, m), 4.20-4.77(1H, m), 6.07(2H, s), 6.88(1H, d), 7.48(1H, d), 7.78(1H, dd), 11.97(1H, br) | $C_{18}H_{25}NO_3 \cdot HCl$ | 63.61<br>63.58 | 7.71<br>7.85 | 4.12<br>4.09 |

Table-12 continued

| | | | | | | | | | |
|---|---|---|---|---|---|---|---|---|---|
| 79 | // | 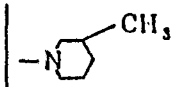 | 169.0-170.0 tetrahydrofuran | 2970, 2940, 2880, 2800-2200, 1670, 1600, 1490, 1440, 1260 | 0.60-4.10(19H, m), 4.17-4.77(1H, m), 6.05(2H, s), 6.87(1H, d), 7.48(1H, d), 7.75(1H, dd), 12.03(1H, br) | $C_{18}H_{25}NO_3 \cdot HCl$ | 63.61<br>63.72 | 7.71<br>7.68 | 4.12<br>4.20 |
| 80 | // | 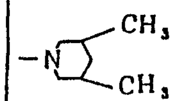 | 186.0-187.0 isopropanol | 2980, 2940, 2880, 2800-2200, 1670, 1600, 1490, 1440, 1260 | 0.57-4.06(21H, m), 4.20-4.70(1H, m), 6.07(2H, s), 6.86(1H, d), 7.45(1H, d), 7.73(1H, dd), 12.17(1H, br) | $C_{10}H_{27}NO_3 \cdot HCl$ | 64.49<br>64.54 | 7.98<br>8.05 | 3.96<br>3.86 |
| 81 | // | 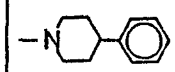 | 135.0-138.0 tetrahydrofuran | 2950, 2910, 2850, 2800-2100, 1650, 1595, 1480, 1430, 1250 | 0.97(3H, t), 1.33-4.20(13H, m), 4.23-4.80(1H, m), 6.03(2H, s), 6.88(1H, d), 7.23(5H, s), 7.50(1H, d), 7.78(1H, dd), 12.10(1H, br) | $C_{23}H_{27}NO_3 \cdot HCl$ | 68.73<br>68.68 | 7.02<br>7.18 | 3.49<br>3.56 |
| 82 | // | 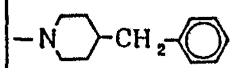 | 142.0-143.0 " | 2950, 2920, 2800-2100, 1655, 1595, 1480, 1440, 1255 | 0.90(3H, t), 1.33-4.10(15H, m), 4.17-4.77(1H, m), 6.02(2H, s), 6.83(1H, d), 6.88-7.40(5H, m), 7.43(1H, d), 7.75(1H, dd), 12.17(1H, br) | $C_{24}H_{20}NO_3 \cdot HCl$ | 69.30<br>69.26 | 7.27<br>7.17 | 3.37<br>3.40 |

Table-12 continued

| | | | | | | | | | |
|---|---|---|---|---|---|---|---|---|---|
| 83 | 〃 | 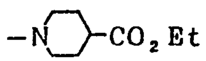 | 86.0 - 88.0<br>〃 | 2950, 2920, 2860, 2800-2100, 1720, 1660, 1600, 1480, 1440, 1260 | 0.68 - 4.75 ( 22H , m ), 6.05 ( 2H , s ), 6.88 ( 1H , d ), 7.48 ( 1H , d ), 7.78 ( 1H , dd ), 12.12 ( 1H , br ) | $C_{20}H_{27}NO_5 \cdot HCl$ | 60.37<br>60.46 | 7.09<br>7.11 | 3.52<br>3.54 |
| 84 | 〃 | 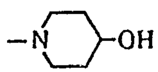 | oil | * 3350, 2950, 2910, 2800-2200, 1660, 1600, 1480, 1430, 1250 | 0.70 - 4.80 ( 18H , m ), 6.07 ( 2H , s ), 6.88 ( 1H , d ), 7.47 ( 1H , d ), 7.70 ( 1H , dd ), 11.03 ( 1H , br ) | $C_{17}H_{23}NO_4 \cdot HCl$ | 59.73<br>59.69 | 7.08<br>6.97 | 4.10<br>4.20 |

* IR(Neat)

Example 85

1-(2,3-Dihydro-5-benzofuranyl)-2-methyl-3-piperidino-1-propanone hydrochloride

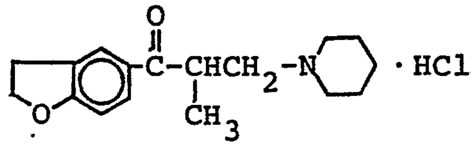

4.0 g of the 5-propionyl-2,3-dihydrobenzofuran prepared in Reference Example 5, 0.91 g of paraformaldehyde and 3.33 g of piperidine hydrochloride were added to 3 ml of isopropanol and the resulting reaction mixture was heated under reflux for 2 hours. After the reaction mixture was allowed to cool, 50 ml of chloroform was added thereto and the resulting mixture was washed three times with 40-ml portions of a saturated aqueous solution of sdium chloride. After the chloroform layer was dried over anhydrous sodium sulfate, the solvent was distilled off under reduced pressure to obtain a residue, which was recrystallized from ethanol to obtain 5.83 g (83 %) of 1-(2,3-dihydro-5-benzofuranyl)-2-methyl-3-piperidino-1-propanone hydrochloride in the form of white crystals, M.P. 193.0 - 194.0°C (dec.).

IR(KBr)$cm^{-1}$: 2960, 2810 - 2200, 1670, 1605, 1590, 1250

NMR($CDCl_3$) δ ppm: 0.93 - 4.93(13H,m), 1.28(3H,d), 3.27(2H,t), 4.68(2H,t), 6.81(1H,d), 7.77 - 8.10(2H,m), 12.03(1H,br)

Elemental analysis for $C_{17}H_{23}NO_2 \cdot HCl$

| | | C | H | N |
|---|---|---|---|---|
| Calcd. | % | 65.90 | 7.81 | 4.52 |
| Found | % | 65.79 | 7.84 | 4.32 |

Example 86

1-(2,3-Dihydro-5-benzofuranyl)-2-methyl-3-piperidino-1-propanone

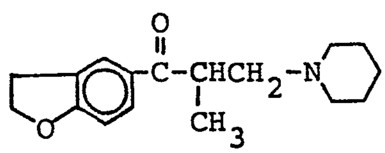

6.0 g of the 1-(2,3-dihydro-5-benzofuranyl)-2-methyl-3-piperidino-1-propanone hydrochloride obtained in Example 85 was dissolved in 75 ml of water and the resulting solution was alkalified by adding 10 ml of a 20 % aqueous solution of

sodium hydroxide. The oily material so separated was extracted with ethyl ether and washed three times with a saturated aqueous solution of sodium chloride. The ether layer was isolated, dried over anhydrous sodium sulfate and then distilled under reduced pressure to remove the solvent. The resulting oily residue was purified by silica gel column chromatography [using an eluent composed of chloroform and ethanol (20:1)] to obtain 4.77 g (90 %) of 1-(2,3-dihydro-5-benzofuranyl)-2-methyl-3-piperidino-1-propanone in the form of an oily material.

IR(Neat)$cm^{-1}$: 2970, 2940, 2860, 2800, 1670, 1600, 1585,1240

NMR($CDCl_3$) $\delta$ ppm: 0.97 - 1.77(6H,m), 1.18(3H,d), 2.13 - 3.90(7H,m), 3.22(2H,t),4.63(2H,t), 6.75(1H,d), 7.67 - 7.97 (2H,m)

Example 87

1-(2,3-Dihydro-5-benzofuranyl)-2-methyl-3-piperidino-1-propanone nitrate

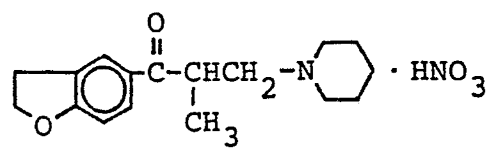

2.0 g of the 1-(2,3-dihydro-5-benzofuranyl)-2-methyl-3-piperidino-1-propanone obtained in Example 86 was dissolved in 30 ml of methanol and the resulting solution was acidified with 30 % nitric acid. Then, the solvent was distilled off under reduced pressure to obtain a residue, to which ethyl ether was added. The resulting mixture was stirred vigorously. The crystals so precipitated were collected by filtration and recrystallized from ethanol to obtain 1.82 g (74 %) of 1-(2,3-dihydro-5-benzofuranyl)-2-methyl-3-piperidino-1-propanone nitrate, M.P. 175.5 - 177.0°C (dec.).

IR(KBr)$cm^{-1}$: 2960, 2870, 2850 - 2200, 1670, 1600, 1585, 1330, 1245

NMR($CDCl_3$) $\delta$ ppm: 1.08 - 4.37(13H,m), 1.24(3H,d), 3.27(2H, t), 4.68(2H,t), 6.83(1H,d), 7.73 - 8.03(2H,m), 10.97(1H,br)

Elemental analysis for $C_{17}H_{23}NO_2 \cdot HNO_3$

| | | C | H | N |
|---|---|---|---|---|
| Calcd. | % | 60.70 | 7.19 | 8.33 |
| Found | % | 60.62 | 7.21 | 8.45 |

Example 88

1-(2,3-Dihydro-5-benzofuranyl)-2-methyl-3-piperidino-1-propanone succinate

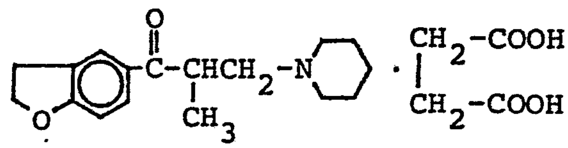

2.0 g of the 1-(2,3-dihydro-5-benzofuranyl)-2-methyl-3-piperidino-1-propanone obtained in Example 86 was dissolved in 30 ml of methanol. To the resulting solution was added a solution of 0.86 g of succinic acid in 40 ml of methanol. After vigorous stirring, the solvent was distilled off under reduced pressure to obtain a residue, to which ethyl ether was added. This residue crystallized when stirred vigorously. The crystals so precipitated were collected by filtration and recrystallized from acetone to obtain 2.15 g (75 %) of 1-(2,3-dihydro-5-benzofuranyl)-2-methyl-3-piperidino-1-propanone succinate, M.P. 110.5 - 111.5°C.

IR(KBr)$cm^{-1}$: 2980, 2950, 2870, 2800 - 2200, 1730, 1675, 1600, 1585, 1555, 1260

NMR($CDCl_3$) $\delta$ ppm: 0.97 - 2.10(6H,m), 1.20(3H,d), 2.48(4H,s), 2.67 - 4.33(7H,m), 3.27(2H,t), 4.63(2H,t), 6.84(1H,d), 7.73 - 8.03(2H,m), 13.62(2H,s)

Elemental analysis for $C_{17}H_{23}NO_2 \cdot C_4H_6O_4$

| | | C | H | N |
|---|---|---|---|---|
| Calcd. | % | 64.43 | 7.47 | 3.58 |
| Found | % | 64.39 | 7.28 | 3.50 |

Example 89

1-(2,3-Dihydro-5-benzofuranyl)-2-ethyl-3-piperidino-1-propanone hydrochloride

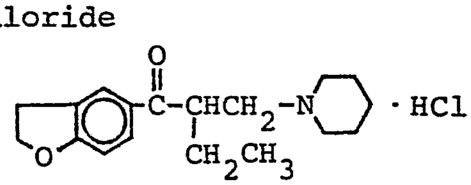

3.0 g of the 5-butyryl-2,3-dihydrobenzofuran prepared in Reference Example 28, 0.63 g of paraformaldehyde and 1.62 g of piperidine were added to 2 ml of isopropanol, followed by adding 1.7 ml of concentrated hydrochloric acid with stirring. The resulting reaction mixture was heated under reflux for 3

hours. After the reaction mixture was allowed to cool, 40 ml of chloroform was added thereto and the resulting mixture was washed three times with 30-ml portions of a saturated aqueous solution of sodium chloride. After the chloroform layer was dried over anhydrous sodium sulfate, the solvent was distilled off under reduced pressure to obtain a residue, which was recrystallized from tetrahydrofuran to obtain 4.14 g (81 %) of 1-(2,3-dihydro-5-benzofuranyl)-2-ethyl-3-piperidino-1-propanone hydrochloride in the form of white crystals, M.P. 181.0 -182.0°C.

IR(KBr)$cm^{-1}$: 2930, 2860, 2800 - 2100, 1675, 1605, 1250

NMR($CDCl_3$) δ ppm: 0.93(3H,t), 1,18 - 4.98(15H,m), 3.28(2H, t), 4.68(2H,t), 6.82(1H,d), 7.78 - 8.18(2H,m), 11.95(1H,br)

Elemental analysis for $C_{18}H_{25}NO_2 \cdot HCl$

| | | C | H | N |
|---|---|---|---|---|
| Calcd. | % | 66.76 | 8.09 | 4.33 |
| Found | % | 67.82 | 8.19 | 4.38 |

Example 90

(+)-1-(2,3-Dihydro-5-benzofuranyl)-2-methyl-3-piperidino-1-propanone hydrochloride

(+)-isomer

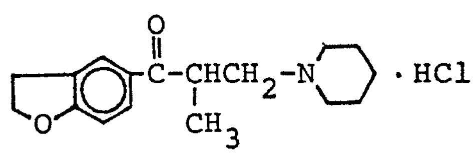

(a) (+)-1-(2,3-Dihydro-5-benzofuranyl)-2-methyl-3-piperidino-1-propanone (-)-N-acetyl-D-α-phenylglycinate

30.0 g of 1-(2,3-dihydro-5-benzofuranyl)-2-methyl-3-piperidino-1-propanone hydrochloride prepared according to the procedure of Example 85 was suspended in 400 ml of water and the resulting suspension was alkalified with a 10 % aqueous solution of sodium hydroxide, followed by vigorous stirring. The oily material so separated was extracted three times with 100-ml portions of benzene. The benzene layer was washed with water, dried over anhydrous sodium sulfate and then distilled under reduced pressure to remove the solvent. The resulting oily residue was dissolved in 100 ml of acetone, and 18.7 g of (-)-N-acetyl-D-α-phenylglycine was added thereto and dissolved therein by the application of heat. After the solution was

allowed to cool, the solvent was distilled off under reduced pressure to obtain an oily residue, to which 200 ml of isopropyl ether was added. While the resulting mixture was being heated under reflux, acetone was added thereto until the oily material was dissolved completely. The resulting solution was allowed to stand in the cold. The crystals so precipitated were collected by filtration and recrystallized three times from a solvent mixture of acetone and isopropyl ether to obtain 12.7 g of (+)-1-(2,3-dihydro-5-benzofuranyl)-2-methyl-3-piperidino-1-propanone (-)-N-acetyl-D-α-phenylglycinate, $[\alpha]_D^{23}$ -41.5° (C=1.0, methanol), M.P. 110.5 - 111.5°C.

IR(KBr)$cm^{-1}$: 3250, 3210, 3070, 2980, 2960, 2940, 2860, 2760 - 2000, 1670, 1605, 1585, 1550

NMR($CDCl_3$) δ ppm: 0.74 - 2.34(6H,m), 1.04(3H,d), 1.99(3H,s), 2.41 - 4.14(7H,m), 3.19(2H,t), 4.60(2H,t), 5.26(1H,d), 6.68 (1H,d), 6.91 - 7.83(8H,m), 13.73(1H,br)

Elemental analysis for $C_{27}H_{34}N_2O_5$

| | | C | H | N |
|---|---|---|---|---|
| Calcd. | % | 69.51 | 7.35 | 6.00 |
| Found | % | 69.40 | 7.41 | 5.93 |

(b) (+)-1-(2,3-Dihydro-5-benzofuranyl)-2-methyl-3-piperidino-1-propanone hydrochloride

9.0 g of the (+)-1-(2,3-dihydro-5-benzofuranyl)-2-methyl-3-piperidino-1-propanone (-)-N-acetyl-D-α-phenylglycinate obtained in (a) was dissolved in 100 ml of water and the resulting solution was alkalified with a 10 % aqueous solution of sodium hydroxide. The oily material so separated was extracted three times with 100-ml portions of benzene. The benzene layer was washed with water, dried over anhydrous sodium sulfate and then concentrated under reduced pressure to a volume of about 30 ml. This solution was acidified by the addition of benzene saturated with hydrogen chloride and then distilled under reduced pressure to remove the solvent. The resulting solid residue was recrystallized from ethanol to obtain 5.0 g of (+)-1-(2,3-dihydro-5-benzofuranyl)-2-methyl-3-piperidino-1-propanone hydrochloride, $[\alpha]_D^{23}$ +42.2°(C=1.0, methanol), M.P. 198.5 - 199.5°C (dec.).

IR(KBr)$cm^{-1}$: 2950, 2800 - 2000, 1665, 1600, 1580

NMR($CDCl_3$) δ ppm: 0.90 - 4.13(12H,m), 1.24(3H,d), 3.25(2H,t), 4.20 - 4.93(1H,m), 4.65(2H,t), 6.79(1H,d), 7.77 - 8.10(2H,m), 12.20(1H,br)

Elemental analysis for $C_{17}H_{23}NO_2 \cdot HCl$

| | | C | H | N |
|---|---|---|---|---|
| Calcd. | % | 65.90 | 7.81 | 4.52 |
| Found | % | 65.99 | 7.73 | 4.50 |

Example 91

(-)-1-(2,3-Dihydro-5-benzofuranyl)-2-methyl-3-piperidino-1-propanone hydrochloride

(-)-isomer 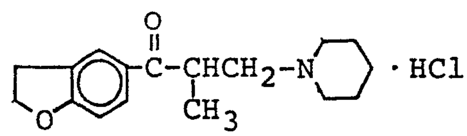

(a) (-)-1-(2,3-Dihydro-5-benzofuranyl)-2-methyl-3-piperidino-1-propanone (+)-N-acetyl-L-α-phenylglycinate

The crystallization and recrystallization mother liquors obtained in Example 90(a) were combined and concentrated under reduced pressure. The resulting residue was dissolved in 250 ml of water and this solution was alkalified with a 10 % aqueous solution of sodium hydroxide. The oily material so separated was extracted three times with 100-ml portions of benzene. The benzene layer was washed with water, dried over anhydrous sodium sulfate and then distilled under reduced pressure to remove the solvent. Thus, there was obtained 15.0 g of an oily material containing a large amount of the (-)-isomer. This oily material was dissolved in 150 ml of acetone, and 10.6 g of (+)-N-acetyl-L-α-phenylglycine was added thereto and dissolved therein by the application of heat. After the solution was allowed to cool, the solvent was distilled off under reduced pressure and the resulting solid residue was recrystallized twice from a solvent mixture of acetone and isopropyl ether (1:1) to obtain 9.0 g of (-)-1-(2,3-dihydro-5-benzofuranyl)-2-methyl-3-piperidino-1-propanone (+)-N-acetyl-L-α-phenylglycinate, $[\alpha]_D^{23}$ +40.9° (C=1.0, methanol), M.P. 110.0 - 111.0°C.

IR(KBr)$cm^{-1}$: 3240, 3200, 3060, 2970, 2950, 2930, 2750 - 2000, 1670, 1600, 1580, 1545

0163537

NMR($CDCl_3$) δ ppm: 0.77 - 2.27(6H,m), 1.03(3H,d), 1.98(3H,s), 2.37 - 4.17(7H,m), 3.17(2H,t), 4.58(2H,t), 5.23(1H,d), 6.65(1H,d), 6.90 - 7.86(8H,m), 13.70(1H,br)

Elemental analysis for $C_{27}H_{34}N_2O_5$

| | | C | H | N |
|---|---|---|---|---|
| Calcd. | % | 69.51 | 7.35 | 6.00 |
| Found | % | 69.56 | 7.32 | 6.15 |

(b) (-)-1-(2,3-Dihydro-5-benzofuranyl)-2-methyl-3-piperidino-1-propanone hydrochloride

4.5 g of the (-)-1-(2,3-dihydro-5-benzofuranyl)-2-methyl-3-piperidino-1-propanone (+)-N-acetyl-L-α-phenylglycinate obtained in (a) was dissolved in 50 ml of water and the resulting solution was alkalified with a 10 % aqueous solution of sodium hydroxide. The oily material so separated was extracted three times with 50-ml portions of benzene. The benzene layer was washed with water, dried over anhydrous sodium sulfate and then concentrated under reduced pressure to a volume of about 50 ml. This solution was acidified by the addition of benzene saturated with hydrogen chloride and then distilled under reduced pressure to remove the solvent. The resulting solid residue was recrystallized from ethanol to obtain 2.7 g of (-)-1-(2,3-dihydro-5-benzofuranyl)-2-methyl-3-piperidino-1-propanone hydrochloride, $[\alpha]_D^{23}$ -42.2° (C=1.0, methanol), M.P. 198.5 - 199.5°C (dec.).

IR(KBr)$cm^{-1}$: 2940, 2800 - 2000, 1665, 1600, 1580

NMR($CDCl_3$) δ ppm: 0.97 - 4.17(12H,m), 1.28(3H,d), 3.27(2H,t), 4.20 - 4.97(1H,m), 4.68(2H,t), 6.83(1H,d), 7.80 - 8.13(2H,m), 12.13(1H,br)

Elemental analysis for $C_{17}H_{23}NO_2{\cdot}HCl$

| | | C | H | N |
|---|---|---|---|---|
| Calcd. | % | 65.90 | 7.81 | 4.52 |
| Found | % | 66.03 | 7.76 | 4.45 |

By the application of the similar procedure as described in Example 85, the compounds of Examples 92 - 124 below were obtained.

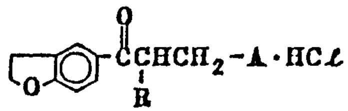

Table-13

| Example No. | R | A | Melting point °C Recrystn. solvent | IR(KBr)$cm^{-1}$ | NMR($CDCl_3$)δ ppm | Molecular formula | Elemental analysis upper row Calcd. lower row Found | | |
|---|---|---|---|---|---|---|---|---|---|
| | | | | | | | C % | H % | N % |
| 92 | $-CH_3$ | 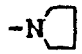 | 186.0-187.5 (dec.) isopropanol-ethylether | 2980, 2950, 2910, 2870, 2820-2100, 1670, 1605, 1590, 1245 | 1.29(3H, d), 1.72-4.96 (11H, m), 3.26(2H, t), 4.67(2H, t), 6.77(1H, d), 7.69-8.09(2H, m), 12.26(1H, br) | $C_{16}H_{21}NO_2 \cdot HCl$ | 64.97<br>65.07 | 7.50<br>7.44 | 4.74<br>4.84 |
| 93 | 〃 | 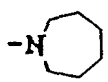 | 173.0-174.0 (dec.) 〃 | 2940, 2870, 2800-2200, 1670, 1605, 1590, 1245 | 0.85-5.05(15H, m), 1.32 (3H, d), 3.28(2H, t), 4.70(2H, t), 6.81(1H, d), 7.75-8.18(2H, m) 11.98(1H, br) | $C_{18}H_{25}NO_2 \cdot HCl$ | 66.76<br>66.85 | 8.09<br>7.92 | 4.33<br>4.48 |
| 94 | 〃 | 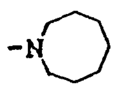 | 160.0-161.0 (dec.) 〃 | 2940, 2870, 2800-2100, 1670, 1605, 1590, 1240 | 0.92-4.95(17H, m), 1.28 (3H, d), 3.28(2H, t), 4.68(2H, t), 6.82(1H, d), 7.75-8.15(2H, m), 11.93(1H, br) | $C_{19}H_{27}NO_2 \cdot HCl$ | 67.54<br>67.65 | 8.35<br>8.42 | 4.15<br>4.18 |
| 95 | $-C_2H_5$ | 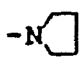 | 185.0-186.0 〃 | 2960, 2860, 2800-2100, 1660, 1605, 1585, 1255 | 0.92(3H, t), 1.27-4.97 (13H, m), 3.28(2H, t), 4.68(2H, t), 6.80(1H, d), 7.77-8.20(2H, m), 12.18(1H, br) | $C_{17}H_{23}NO_2 \cdot HCl$ | 65.90<br>65.86 | 7.81<br>7.78 | 4.52<br>4.68 |

Table-13 continued

| | | | | | | | | | |
|---|---|---|---|---|---|---|---|---|---|
| 96 | 〃 | 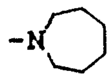 | 148.0-150.0 tetrahydrofuran | 2940, 2910, 2840, 2770-2100, 1650, 1600, 1240 | 0.92(3H, t), 1.30-4.97 (17H, m), 3.30(2H, t), 4.70(2H, t), 6.85(1H, d), 7.87-8.20(2H, m), 12.12(1H, br) | $C_{19}H_{27}NO_2 \cdot HCl$ | 67.54<br>67.63 | 8.35<br>8.52 | 4.15<br>4.27 |
| 97 | 〃 | 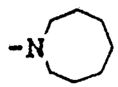 | 117.0-119.0<br>〃 | 2950, 2920, 2850, 2800-2100, 1655, 1605, 1250 | 0.88(3H, t), 1.17-4.90 (19H, m), 3.22(2H, t), 4.63(2H, t), 6.75(1H, d), 7.73-8.13(2H, m), 12.00(1H, br) | $C_{20}H_{29}NO_2 \cdot HCl$ | 68.26<br>68.28 | 8.59<br>8.43 | 3.98<br>4.00 |
| 98 | $-n-C_3H_7$ | 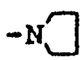 | 160.5-161.5 (dec.) isopropanol-ethylether | 2960, 2930, 2880, 2780-2000, 1660, 1600, 1580, 1255 | 0.65-5.02(18H, m), 3.27 (2H, t), 4.68(2H, t), 6.81(1H, d), 7.82-8.22 (2H, m), 12.38(1H, br) | $C_{18}H_{25}NO_2 \cdot HCl$ | 66.76<br>66.86 | 8.09<br>8.15 | 4.33<br>4.27 |
| 99 | 〃 | 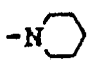 | 151.5-152.5 tetrahydrofuran | 2960, 2940, 2870, 2800-2100, 1660, 1600, 1240 | 0.62-4.91(20H, m), 3.28 (2H, t), 4.68(2H, t), 6.81(1H, d), 7.78-8.15 (2H, m), 12.10(1H, br) | $C_{19}H_{27}NO_2 \cdot HCl$ | 67.54<br>67.65 | 8.35<br>8.46 | 4.15<br>4.19 |
| 100 | 〃 | 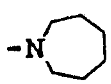 | 159.0-160.0 isopropanol-ethylether | 2960, 2940, 2880, 2800-2200, 1655, 1600, 1240 | 0.62-4.95(22H, m), 3.28 (2H, t), 4.68(2H, t), 6.82(1H, d), 7.78-8.15 (2H, m), 12.18(1H, br) | $C_{20}H_{29}NO_2 \cdot HCl$ | 68.26<br>68.34 | 8.59<br>8.68 | 3.98<br>3.90 |

Table-13 continued

| | | | | | | | | | |
|---|---|---|---|---|---|---|---|---|---|
| 101 | $-iso-C_3H_7$ | 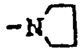 | 181.0-182.0<br>" | 2980, 2900, 2800-2100, 1670, 1605, 1240 | 0.85(3H, d), 1.08(3H, d), 0.52-4.18(11H, m), 3.28(2H, t), 4.22-4.98 (1H, m), 4.68(2H, t), 6.83(1H, d), 7.82-8.22 (2H, m), 12.32(1H, br) | $C_{18}H_{25}NO_2 \cdot HCl$ | 66.76<br>66.74 | 8.09<br>8.14 | 4.33<br>4.24 |
| 102 | " | 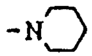 | 181.5-182.5<br>tetranydrofuran | 2980, 2890, 2800-2100, 1670, 1605, 1250 | 0.45-4.95(14H, m), 0.81 (3H, d), 1.18(3H, d), 3.32(2H, t), 4.68(2H, t), 6.78(1H, d), 7.75-8.18(2H, m), 11.95(1H, br) | $C_{19}H_{27}NO_2 \cdot HCl$ | 67.54<br>67.63 | 8.35<br>8.49 | 4.15<br>4.08 |
| 103 | $-n-C_4H_9$ | 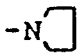 | 179.0-180.0<br>(dec.)<br>isopropanol-ethylether | 2960, 2940, 2880, 2800-2100, 1660, 1600, 1245 | 0.58-4.95(20H, m), 3.27 (2H, t), 4.68(2H, t), 6.81(1H, d), 7.78-8.18 (2H, m), 12.35(1H, br) | $C_{19}H_{27}NO_2 \cdot HCl$ | 67.54<br>67.69 | 8.35<br>8.40 | 4.15<br>4.09 |
| 104 | " | 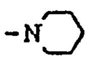 | 180.0-181.0<br>(dec.)<br>" | 2960, 2940, 2880, 2800-2200, 1650, 1600, 1240 | 0.55-4.95(22H, m), 3.28 (2H, t), 4.68(2H, t), 6.81(1H, d), 7.75-8.22 (2H, m), 12.07(1H, br) | $C_{20}H_{29}NO_2 \cdot HCl$ | 68.26<br>68.33 | 8.59<br>8.62 | 3.98<br>4.05 |
| 105 | $-iso-C_4H_9$ | 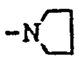 | 173.0-174.0<br>tetranydrofuran | 2930, 2750-2000, 1650, 1595, 1575, 1250 | 0.70-4.97(14H, m), 0.88 (3H, d), 1.02(3H, d), 3.28(2H, t), 4.70(2H, t), 6.83(1H, d), 7.83-8.20(2H, m), 12.47 (1H, br) | $C_{19}H_{27}NO_2 \cdot HCl$ | 67.54<br>67.43 | 8.35<br>8.41 | 4.15<br>4.05 |

Table-13 continued

| | | | | | | | | | |
|---|---|---|---|---|---|---|---|---|---|
| 106 | $-iso-C_4H_9$ | 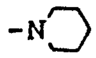 | 156.0-157.0 tetrahydrofuran -ethylether | 2950, 2850, 2750-2200, 1655, 1600, 1580, 1260, 1240 | 0.50-4.93(16H, m), 0.92 (3H, d), 1.05(3H, d), 3.28(2H, t), 4.68(2H, t), 6.82(1H, d), 7.80-8.20(2H, m), 12.20(1H, br) | $C_{20}H_{29}NO_2 \cdot HCl$ | 68.26<br>68.38 | 8.59<br>8.64 | 3.98<br>4.03 |
| 107 | $-n-C_5H_{11}$ | 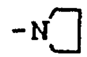 | 164.0-165.0 tetranydrofuran | 2950, 2920, 2850, 2770-2200, 1650, 1600, 1250 | 0.58-4.98(22H, m), 3.30 (2H, t), 4.70(2H, t), 6.86(1H, d), 7.88-8.22 (2H, m), 12.45(1H, br) | $C_{20}H_{29}NO_2 \cdot HCl$ | 68.26<br>68.19 | 8.59<br>8.47 | 3.98<br>3.83 |
| 108 | 〃 | 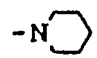 | 174.0-175.0 " | 2910, 2840, 2790-2200, 1645, 1600, 1240 | 0.52-4.92(24H, m), 3.25 (2H, t), 4.65(2H, t), 6.79(1H, d), 7.78-8.12 (2H, m), 12.13(1H, br) | $C_{21}H_{31}NO_2 \cdot HCl$ | 68.93<br>69.04 | 8.81<br>8.89 | 3.83<br>3.96 |
| 109 | $-n-C_6H_{13}$ | 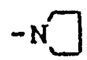 | 131.0-133.0 " | 2940, 2910, 2850, 2770-2200, 1650, 1600, 1245 | 0.43-4.93(24H, m), 3.28 (2H, t), 4.68(2H, t), 6.82(1H, d), 7.83-8.20 (2H, m), 12.48(1H, br) | $C_{21}H_{31}NO_2 \cdot HCl$ | 68.93<br>68.86 | 8.81<br>8.85 | 3.83<br>3.90 |
| 110 | 〃 | 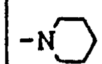 | 127.0-129.0 tetrahydrofuran -ethylether | 2940, 2910, 2840, 2790-2200, 1650, 1600, 1240 | 0.52-4.95(26H, m), 3.28 (2H, t), 4.68(2H, t), 6.84(1H, d), 7.82-8.18 (2H, m), 12.13(1H, br) | $C_{22}H_{33}NO_2 \cdot HCl$ | 69.54<br>69.65 | 9.02<br>8.94 | 3.69<br>3.75 |
| 111 | 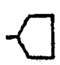 | 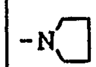 | 174.0-176.0 tetrahydrofuran | 2950, 2890, 2850, 2770-2000, 1660, 1600, 1580, 1245 | 0.85-5.02(20H, m), 3.30 (2H, t), 4.68(2H, t), 6.79(1H, d), 7.78-8.32 (2H, m), 12.38(1H, br) | $C_{20}H_{27}NO_2 \cdot HCl$ | 68.65<br>68.58 | 8.07<br>8.12 | 4.00<br>3.97 |

| | | | | | | | | | |
|---|---|---|---|---|---|---|---|---|---|
| 112 | 〃 | 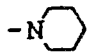 | 178.0-179.0<br>" | 2950,2930,<br>2890,2860,<br>2750-2100,<br>1655,1600,<br>1250 | 0.90-4.97(22H,m),3.28<br>(2H,t),4.68(2H,t),<br>6.84(1H,d),7.83-8.23<br>(2H,m),12.20(1H,br) | $C_{21}H_{29}NO_2 \cdot HCl$ | 69.31<br>69.42 | 8.31<br>8.38 | 3.85<br>3.69 |
| 113 | 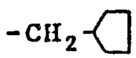 | 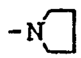 | 191.0-193.0<br>(dec.)<br>〃 | 2940,2900,<br>2860,2750-<br>2100,1655,<br>1600,1580,<br>1255 | 0.73-4.90(22H,m),3.27<br>(2H,t),4.67(2H,t),<br>6.83(1H,d),7.93-8.27<br>(2H,m),12.53(1H,br) | $C_{21}H_{29}NO_2 \cdot HCl$ | 69.31<br>69.50 | 8.31<br>8.26 | 3.85<br>3.87 |
| 114 | 〃 | 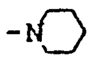 | 166.0-168.0<br>(dec.)<br>" | 2920,2850,<br>2770-2100,<br>1640,1595,<br>1240 | 0.77-4.93(24H,m),3.28<br>(2H,t),4.68(2H,t),<br>6.84(1H,d),7.83-8.27<br>(2H,m),12.23(1H,br) | $C_{22}H_{31}NO_2 \cdot HCl$ | 69.91<br>70.01 | 8.53<br>8.47 | 3.71<br>3.69 |
| 115 | 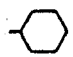 | 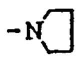 | 188.0-189.0<br>(dec.)<br>isopropanol-<br>ethylether | 2930,2860,<br>2770-2000,<br>1660,1600,<br>1240 | 0.55-4.90(22H,m),3.30<br>(2H,t),4.70(2H,t),<br>6.83(1H,d),7.68-8.12<br>(2H,m),12.37(1H,br) | $C_{21}H_{29}NO_2 \cdot HCl \cdot 1/2(Et)_2O$ | 68.89<br>68.95 | 8.80<br>8.83 | 3.49<br>3.57 |
| 116 | 〃 | 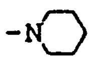 | 185.0-186.0<br>(dec.)<br>" | 2940,2860,<br>2780-2000,<br>1660,1600,<br>1245 | 0.53-4.97(24H,m),3.30<br>(2H,t),4.69(2H,t),<br>6.83(1H,d),7.72-8.13<br>(2H,m),12.03(1H,br) | $C_{22}H_{31}NO_2 \cdot HCl \cdot 1/2(Et)_2O$ | 69.46<br>69.53 | 8.99<br>8.85 | 3.38<br>3.50 |
| 117 | $-CH_3$ | 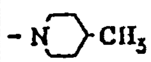 | 192.0-193.5<br>(dec.)<br>ethanol-<br>tetrahydrofuran | 2960,2930,<br>2880,2800-<br>2200,1670,<br>1600,1245 | 0.70-4.97(15H,m),1.30<br>(3H,d),3.30(2H,t),<br>4.70(2H,t),6.83(1H,<br>d),7.77-8.17(2H,m),<br>12.03(1H,br) | $C_{18}H_{25}NO_2 \cdot HCl$ | 66.76<br>66.85 | 8.09<br>8.16 | 4.33<br>4.28 |

Table-13 continued

| | | | | | | | | | |
|---|---|---|---|---|---|---|---|---|---|
| 118 | 〃 | 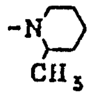 | oil | ** 2950, 2830-2200, 1670, 1600, 1590, 1250 | 1.00-5.03(15H, m), 1.29 (3H, d), 3.27(2H, t), 4.68(2H, t), 6.81(1H, d), 7.77-8.17(2H, m), 11.83(1H, br) | $C_{18}H_{25}NO_2 \cdot HCl \cdot H_2O$ | 63.24<br>63.31 | 8.26<br>8.33 | 4.10<br>4.19 |
| 119 | 〃 | 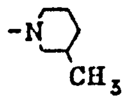 | 185.0-186.0 (dec.) ethanol-tetrahydrofuran | 2970, 2890, 2800-2200, 1670, 1610, 1590, 1250 | 0.66-5.00(12H, m), 0.84 (3H, d), 1.34(3H, d), 3.32(2H, t), 4.73(2H, t), 6.88(1H, d), 7.87-8.20(2H, m), 12.10(1H, br) | $C_{18}H_{25}NO_2 \cdot HCl$ | 66.76<br>66.72 | 8.09<br>8.00 | 4.33<br>4.51 |
| 120 | 〃 | 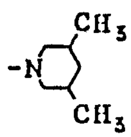 | 178.0-179.0 isopropanol | 2970, 2930, 2880, 2800-2200, 1670, 1605, 1585, 1250 | 0.50-4.97(11H, m), 0.82 (3H, d), 0.95(3H, d), 1.29(3H, d), 3.28(2H, t), 4.70(2H, t), 6.83(1H, d), 7.77-8.13(2H, m), 12.17(1H, br) | $C_{19}H_{27}NO_2 \cdot HCl \cdot 1/2H_2O$ | 65.79<br>65.70 | 8.43<br>8.28 | 4.04<br>4.17 |
| 121 | 〃 | 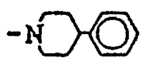 | 174.0-176.0 (dec.) 〃 | 2970, 2930, 2800-2200, 1655, 1600, 1580, 1250 | 1.33(3H, d), 1.57-4.97 (12H, m), 3.25(2H, t), 4.67(2H, t), 6.81(1H, d), 7.23(5H, s), 7.80-8.17(2H, m), 12.37(1H, br) | $C_{23}H_{27}NO_2 \cdot HCl$ | 71.58<br>71.66 | 7.31<br>7.42 | 3.63<br>3.70 |

Table-13 continued

| | | | | | | | | | |
|---|---|---|---|---|---|---|---|---|---|
| 122 | 〃 | 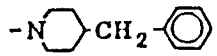 | 199.0-201.0 (dec.) ethanol | 2950, 2940, 2800-2200, 1660, 1600, 1245 | 1.26(3H, d), 1.47-4.93 (14H, m), 3.23(2H, t), 4.65(2H, t), 6.79(1H, d), 6.83-7.47(5H, m), 7.77-8.10(2H, m), 11.77 (1H, br) | $C_{24}H_{29}NO_2 \cdot HCl$ | 72.07<br>72.18 | 7.56<br>7.62 | 3.50<br>3.48 |
| 123 | 〃 | 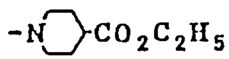 | 195.0-196.0 isopropanol | 2980, 2920, 2800-2200, 1720, 1660, 1600, 1585, 1250 | 0.93-1.53(6H, m), 1.63 -5.07(14H, m), 3.23(2H, t), 4.67(2H, t), 6.79(1H, d), 7.77-8.13(2H, m), 11.83(1H, br) | $C_{20}H_{27}NO_4 \cdot HCl$ | 62.90<br>62.85 | 7.39<br>7.20 | 3.67<br>3.71 |
| 124 | 〃 | 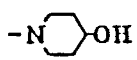 | 202.0-204.0 ethanol | 3320, 2920, 2870, 2780-2200, 1655, 1605, 1585, 1250 | ***1.31(3H, d), 1.57-5.00 (14H, m), 4.67(2H, t), 6.83(1H, d), 7.77-8.10 (2H, m) | $C_{17}H_{23}NO_3 \cdot HCl$ | 62.67<br>62.73 | 7.43<br>7.55 | 4.30<br>4.27 |

*NMR(DMSO-$d_6$+$CD_3OD$), **IR(Neat), ***NMR($CDCl_3$+$CD_3OD$)

What is claimed is:

1. 1-Propanone derivatives represented by the formula:

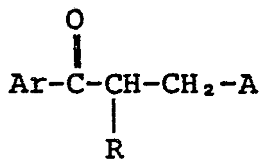

wherein Ar is a 4-cycloalkylphenyl, 3,4-methylenedioxyphenyl or 2,3-dihydro-5-benzofuranyl group, R is a straight-chain, branched or cyclic alkyl group having 1 to 6 carbon atoms or a cyclopentylmethyl group, and A is an unsubstituted or substituted 1-pyrrolidinyl, piperidino, hexahydro-1*H*-azepin-1-yl or octahydroazocin-1-yl group, and physiologically acceptable acid addition salts thereof.

2. Derivatives as claimed in claim 1 wherein Ar is a 4-cycloalkylphenyl group, and physiologically acceptable acid addition salts thereof.

3. Derivatives as claimed in claim 1 or 2 wherein the 4-cycloalkylphenyl group is 4-cyclopropylphenyl, and physiologically acceptable acid addition salts thereof.

4. Derivatives as claimed in claim 1 wherein Ar is a 3,4-methylenedioxyphenyl group, and physiologically acceptable acid addition salts thereof.

5. Derivatives as claimed in claim 1 wherein Ar is a 2,3-dihydro-5-benzofuranyl group, and physiologically acceptable acid addition salts thereof.

6. 1-(4-Cyclopropylphenyl)-2-methyl-3-(1-pyrrolidinyl)-1-propanone hydrochloride.

7. 1-(4-Cyclopropylphenyl)-2-methyl-3-piperidino-1-propanone hydrochloride.

8. 1-(4-Cyclopropylphenyl)-2-methyl-3-(hexahydro-1*H*-azepin-1-yl)-1-propanone hydrochloride.

9. 1-(4-Cyclopropylphenyl)-2-ethyl-3-(1-pyrrolidinyl)-1-propanone hydrochloride.

10. 1-(4-Cyclopropylphenyl)-2-ethyl-3-piperidino-1-propanone hydrochloride.

11. 1-(4-Cyclopropylphenyl)-2-isopropyl-3-piperidino-1-propanone hydrochloride.

12. 2-Methyl-1-(3,4-methylenedioxyphenyl)-3-(1-pyrrolidinyl)-1-propanone hydrochloride.

13. 2-Methyl-1-(3,4-methylenedioxyphenyl)-3-piperidino-1-propanone hydrochloride.

14. 2-Methyl-1-(3,4-methylenedioxyphenyl)-3-(hexahydro-1*H*-azepin-1-yl)-1-propanone hydrochloride.

15. 2-Ethyl-1-(3,4-methylenedioxyphenyl)-3-(1-pyrrolidinyl)-1-propanone hydrochloride.

16. 2-Ethyl-1-(3,4-methylenedioxyphenyl)-3-piperidino-1-propanone hydrochloride.

17. 1-(3,4-Methylenedioxyphenyl)-2-n-propyl-3-piperidino-1-propanone hydrochloride.

18. 1-(2,3-Dihydro-5-benzofuranyl)-2-methyl-3-(1-pyrrolidinyl)-1-propanone hydrochloride.

19. 1-(2,3-Dihydro-5-benzofuranyl)-2-methyl-3-piperidino-1-propanone hydrochloride.

20. 1-(2,3-Dihydro-5-benzofuranyl)-2-methyl-3-(hexahydro-1*H*-azepin-1-yl)-1-propanone hydrochloride.

21. 1-(2,3-Dihydro-5-benzofuranyl)-2-ethyl-3-(1-pyrrolidinyl)-1-propanone hydrochloride.

22. 1-(2,3-Dihydro-5-benzofuranyl)-2-ethyl-3-piperidino-1-propanone hydrochloride.

23. 1-(2,3-Dihydro-5-benzofuranyl)-2-ethyl-3-(hexahydro-1*H*-azepin-1-yl)-1-propanone hydrochloride.

24. A pharmaceutical composition having central muscle relaxant activity which comprises a compound as claimed in any of claims 1 to 23 and a pharmaceutically acceptable carrier.

European Patent Office

# EUROPEAN SEARCH REPORT

0163537
Application number

EP 85303804.0

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.4) |
|---|---|---|---|
| A | EP - A1 - 0 105 632 (DAINIPPON PHARMACEUTICAL)<br>* Abstract; page 2, line 1 - page 3, line 15 *<br>-- | 1,24 | C 07 D 295/10<br>C 07 D 317/58<br>C 07 D 307/79<br>C 07 D 405/06<br>A 61 K 31/395 |
| A | GB - A - 1 213 963 (RICHTER GEDEON VEGYESZETI GYAR)<br>* Page 1, lines 15-33 *<br>-- | 1,24 | |
| D,A | US - A - 4 181 803 (MORITA et al.)<br>* Claim 1; abstract *<br>-- | 1,24 | |
| D,A | THE JOURNAL OF THE AMERICAN CHEMICAL SOCIETY, vol. 71, 1949 Easton<br>J.J. DENTON et al. "Antispasmodics. I. Substituted β-amino ketones"<br>pages 2048-2050<br>* Page 2049, tabel I, numbers XI, XV; summary *<br>-- | 1,4, 24 | **TECHNICAL FIELDS SEARCHED (Int. Cl.4)**<br>C 07 D 295/00<br>C 07 D 317/00<br>C 07 D 307/00 |
| A | EP - A1 - 0 061 149 (MITSUBISHI CHEMICAL INDUSTRIES)<br>* Examples 1,41-45, 50-55 *<br>---- | 1,4 | |

The present search report has been drawn up for all claims

| Place of search | Date of completion of the search | Examiner |
|---|---|---|
| VIENNA | 16-08-1985 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82